# EUROPEAN PATENT SPECIFICATION

(11) **EP 1 465 558 B1**
(45) Date of publication and mention of the grant of the patent: **01.07.2009**
(21) Application number: 02793301.9
(22) Date of filing: 10.12.2002
(51) Int. Cl.: A61F 2/44, A61L 27/24, A61L 27/50

(54) **USE OF CROSS-LINKED COLLAGEN FOR INTERVERTEBRAL DISC TREATMENT**
VERWENDUNG VON VERNETZTEM KOLLAGEN ZUR BEHANDLUNG VON BANDSCHEIBEN
UTILISATION DE COLLAGÈNE RÉTICULÉ POUR LE TRAITEMENT DE DISQUES INTERVERTEBRAUX

(30) Priority: 10.12.2001 US 337145 P
(43) Date of publication of application: 13.10.2004
(73) Proprietor: Colbar Lifescience Ltd., Ramat Hasharon 47203 (IL)
(72) Inventor: PITARU, Shahar, 53303 Givatayim (IL); NOFF, Matitiau, 69620 Tel Aviv (IL)
(74) Representative: Gervasi, Gemma
(86) International application number: PCT/IL2002/000997
(87) International publication number: WO 2003/049669

(56) References cited:
- WO-A1-96/31157
- WO-A2-01/79342
- US-A- 4 971 954
- US-A- 5 108 438
- US-A- 5 955 438
- US-A1- 2002 019 516
- US-A1- 2002 042 652

## Description

### FIELD OF THE INVENTION

The present invention relates generally to preparations and their use for treating intervertebral disc pathologies, and more specifically to preparations and implants including collagen, and cross-linked collagen and methods and devices for their introduction into mammalian intervertebral discs.

### BACKGROUND OF THE INVENTION

Intervertebral discs are semi-elastic discs, which lie between the rigid bodies of adjacent vertebrae. Intervertebral discs form about one-fourth the length of the vertebral column.

Intervertebral discs are composed of two major anatomic zones, a peripheral part, the *annulus fibrosus* and a central part, the *nucleus pulposus.* The annulus fibrosus is composed of laminated fibrous tissue wrapped around the gelatinous nucleus pulposus. The parts of the vertebrae adjacent the inter-vertebral disc surfaces are called end plates The nucleus pulposus of the Intervertebral discs are not vascularized and therefore depend on diffusion of nutrients through the endplates.

The annulus fibrosus is composed of laminae of fibrous tissue, in which collagen fibers are arranged in concentric layers or sheets also known as lamellae. The outer or peripheral layers of the annulus include type I collagen. In comparison, the inner annular layers lying next to the nucleus pulposus are referred to as the *transitional zone* and include fibrocartilaginous material. The collagen bundles in the laminae of the annulus fibrosus pass obliquely between adjacent vertebral bodies, and their inclination is reversed in alternate sheets. The collagen fibers' varying angles accommodate to all the angles of force that can be applied to the disc. The nucleus pulposus includes a lattice framework of collagen embedded in a highly hydrated gelatinous mass containing cartilage cells known as chondrocytes and other biochemicals such as mucoproteins and proteoglycans. The nucleus pulposus contains Type II collagen.

Functionally, the intervertebral discs performs two important, but somewhat conflicting roles. They maintain spinal column stability while providing the column with necessary flexibility. Without intervertebral discs, the human spine is unable to bend and its function is greatly impaired.

Numerous nerves cross outside the spinal column through openings between the vertebrae, as well as directly down the spinal cord. Therefore, intervertebral discs have to keep the vertebral bodies separated so as to hold the foramen space open and provide adequate spinal column stability. The intervertebral discs also function as shock absorbers. The physical characteristics of the intervertebral discs permit them to serve as shock absorbers when the load on the vertebral column is suddenly increased. The semi fluid nature of the nucleus pulposus allows the discs to change shape and permits one vertebra to rock forward or backward on another, as in flexion and extension of the vertebral column. The resistance of the discs to compression forces is substantial.

Nevertheless, the discs are vulnerable to sudden shocks, trauma or strain, particularly if the vertebral column is flexed or if the discs are undergoing degenerative changes, which may result in loosing of disc height and disc herniation. Unfortunately, the intervertebral discs' resilience is gradually lost with advancing age. In old age the discs are thin and less elastic. The changes accounting to thinning and elasticity loss occur in the annulus as well as in the nucleus. With advancing age the collagen fibers of the annulus fibrosus degenerate, the annulus shows coarsened and hyalinized fibers and fissuring of the lamellae, and the annulus capability to contain the nucleus pulposus decreases. In parallel, the water content of the nucleus pulposus diminishes with aging and the hydrogel may be gradually replaced by fibrocartilage. These age related changes may result in various forms of intervertebral disc pathologies.

In herniated discs, part or all of the soft gelatinous material comprising the nucleus pulposus is forced through a weakened or ruptured part of the annulus fibrosus, which may result, inter alia, in back pain and nerve root irritation (radiculopathy) due to the resulting nerve root or spinal cord compression.

The discs most commonly affected in intervertebral disc disease (IVD) or disc herniation (disc rupture) are those in spinal regions where a mobile part of the column joins a relatively immobile part, that is, the cervico-thoracic junction and the lumbo-sacral junction.

The role of the diseased intervertebral disc itself as a pain generator was not widely recognized until 1990's and many surgeons emphasize the nerve root compression component as the major factor to be addressed by surgical treatment. Nevertheless, the intervertebral disc as a pain generator is slowly gaining wider acceptance *(*Spine 20:665-669, 1995, Spine 20:1878-1883, 1995) and many people advocate removal of disc with or without herniation and nerve root compression *(*Orthopaedics 14:447-451, 1991, Spine 17: 831-833, 1992). Major forms of intervertebral disc disease or pathology include, inter alia, annular lamellar ruptures, disc herniation, ruptured posterior longitudinal ligament, annulus fibrosus (extruded disc material), and degeneration of the intervertebral disc.

The term degeneration of the intervertebral disc may imply an inevitable progression that is characteristic of wear-and-tear-associated conditions. Modern research on human tissue has, however, shown that this is not the case. The disruption of the micro-anatomy that is described as degeneration is an active process, which is probably regulated by locally produced cytokines. In disc degeneration, there is a disruption of the nucleus pulposus, including changes in the proportion and types of proteoglycans and collagens, a reduction in the number of chondrocytes, and the formation of permeative 'slit-like' spaces within the nucleus pulposus. Often, there is disruption of the collagen fiber arrays in the annulus fibrosus, traumatic damage to the disc end plate, and ingrowth of blood vessels and nerves into the nucleus pulposus. From our current understanding of the biology of connective tissues, it seems probable that alterations in the function of local cells are central to these events. (Tony J. Freemont, Christine LeMaitre, Alex Watkins and Judith A. Hoyland Histological sections of normal and degenerate intervertebral discs (IVDs) Expert Reviews in Molecular Medicine, 29 March 2001)

The composition of collagenous tissues changes naturally with an individual's age. *(Erdal CO* 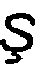 *KUN, Tuncer SÜZER, Gülgün OKTAY, Zeynep TOKGÖZ, Mehmet H. KÖSEO* 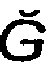 *LU* COLLAGEN CONTENTS IN LUMBAR INTERVERTEBRAL DISC PROTRUSIONS AND FREE FRAGMENTS Journal of Neurological Sciences (Turkish) ISSN 1302-1664, 17: 3 , 2000)

The reduction in the number of chondrocytes that typifies IVD degeneration has been ascribed to apoptosis (Gruber, H.E. and Hanley, E.N., Jr., "Analysis of aging and degeneration of the human intervertebral disc. Comparison of surgical specimens with normal controls", Spine 23, 751-757,1998). In articular cartilage of patients who have osteoarthritis, a disorder with some similarities to disc degeneration, chondrocyte apoptosis has been associated with the local production of nitric oxide. It is not known if the same process occurs in IVDs.

The cell biology of chondrocytes in degenerate IVDs is altered profoundly compared with chondrocytes from age and sex-matched controls. Normal discal chondrocytes are characterised by the expression of type II collagen and proteoglycans (Chelberg, M.K. et al. "Identification of heterogeneous cell populations in normal human intervertebral disc.", J. Anat. 186, 43-53,1995). In degeneration, there is a net increase in matrix-degrading enzyme activity over natural inhibitors of such activity, which leads to the loss of discal matrix (Kanemoto, M. et al. "Immunohistochemical study of matrix metalloproteinase-3 and tissue inhibitor of metalloproteinase-1 human intervertebral discs", Spine 21, 1-8, 1996).

Although the normal adult IVD is avascular and aneural, nerves and blood vessels grow into diseased IVDs (Yasuma, T., Arai, K. and Yamauchi, Y., "The histology of lumbar intervertebral disc herniation - the significance of small blood vessels in the extruded tissue.", Spine 18, 1761-1765, 1993). One avenue of investigation has been the local production of angiogenic and neurogenic molecules within degenerate IVDs. Expression of the potent angiogenic factor vascular endothelial growth factor (VEGF) (Tolonen, J. et al., "Platelet-derived growth factor and vascular endothelial growth factor expression in disc herniation tissue: an immunohistochemical study.", Eur Spine J., 6, 63-69, 1997).

Lumbar spine decompression is a commonly performed procedure that is indicated for herniated nucleus pulposus. Current methods for lumbar spine decompression may be divided into open surgery and percutaneous surgical techniques. Open surgery techniques include lamina removal and fusion techniques. Percutaneous techniques include Laser Disc Decompression (PLDD) and electrosurgical spine treatment. Basically, surgery cannot repair the disc itself. What it can do is provide more room for the herniated disc to bulge in, thereby reducing pressure on the nerves which may reduce pain.

Surgical removal of an intervertebral disc or portions thereof may necessitate additional surgical procedures in cases where instability between spinal vertebrae is present. Spinal fusion or interbody fixation may be used in such cases. The advantages of interbody fixation include direct removal of the dysfunctional disc and preservation or restoration of the disc height. Maintenance of the disc height, is important to achieve significant increase in the neuroforamen volume.

One approach to stabilizing the vertebrae, termed spinal fusion, is to insert an interbody graft or implant into the space vacated by the degenerative disc. For example, in posterior lumbar interbody fusion (PLIF), two adjacent vertebral bodies are fused together by removing the affected disc and inserting an implant that would allow for bone to grow between the two vertebral bodies to bridge the gap left by the disc removal. A small amount of bone is grafted from other portions of the body, such as the hip, and packed into the implants. This allows the bone to grow through and around the implant, fusing the vertebral bodies and alleviating the pain.

One of the negative aspects of spinal fusion is that there is no movement between the two fused vertebra. The adjacent segments are extra-loaded when the spinal column bends. The result is that adjacent discs will degenerate faster. Artificial discs have also been used. The artificial disc is designed to replace the entire disc with or without leaving some of the annulus. Most artificial disc designs require removal of the endplates and fixation of the superior and inferior surfaces of the implant to the vertebral bodies. The main benefit of replacing the entire disc is that the disc is consequently less dependent on the integrity of the annulus and the stage of degeneration. Conceptually, artificial discs can be used in patients with disc degeneration at any stage of progression. Because of the added cost and high risk involved in implanting such a device, however, in practice its use can often be justified only in patients with more severe disc degeneration.

For flexibility, either the material must be elastic itself or the design must have elastic characteristics at least in one direction (preferably in multiple directions). On the other hand, because the implant must maintain a firm fixation to the vertebrae, a hard material such as metal must often be used for the superior and inferior surfaces of the device. Fixation is often achieved by one or a combination of the following mechanisms: 1) anchoring through one or several pegs or posts inserted into the vertebrae; 2) physical interfacing via a threaded surface; 3) promotion of bone ingrowth by means of a porous surface; or 4) fixation with screws through a side wing extending from the plate. Another option is to replace solely the nucleus. The prosthetic disc nucleus (PDN), for example, replaces the nucleus with two mini "pillows".

US patent 5,108,438 discloses a prosthetic intervertebral disc which may be implanted in the human skeleton, and which may act as a scaffold for regrowth of intervertebral disc material. The prosthetic disc includes a dry, porous, volume matrix of biocompatible and bioresorbable fibers which may be interspersed with glyscosaminoglycan molecules

Laminectomy is an operation performed to relieve pressure on one or more nerve roots. The most common laminectomy is lumbar laminectomy, which is performed on the lower spine. Pressure on a nerve root in the lower spine, often called nerve root compression, causes back and leg pain. In this operation the surgeon typically reaches the lumbar spine through a small midline posterior incision. A portion of the lamina is removed to expose the compressed nerve root(s). According to the pathology, the laminectomy is performed on one side or bilaterally. Pressure is relieved by removal of the source of compression such as part of the herniated disc, a disc fragment, a tumor, or a bone spur.

Laminotomy is a less invasive, procedure which may be regarded as a refined version of laminectomy. In laminotomy only a small part of the lamina directly surrounding the affected disc is removed. There is growing evidence that laminotomy is superior. It is believed that the less bone that is removed, the more strong and stabile the remaining structure is. While performing those procedures will often relieve symptoms initially, there is a high incidence of subsequent complications, often worse than the original problem, because of the resulting spinal instability. Laminectomy as well as laminotomy may include an insertion of a space maintainer between the vertebrae.

US patent 6,283,968 discloses a posterior approach *laminectomy* procedure for placing a prosthesis within the intradiscal space between adjacent vertebrae.

In laminoplasty the back of the spine is exposed but instead of the bony structures being removed as done in laminectomy and laminotomy, they are being weakened and bent outwards thus opening the canal and providing more room for the spinal cord. The problem is how to stabilize the lamina in this new position.

Several techniques are used for lamina stabilization. One way of stabilizing the lamina is to take a bone graft from the *Illium* in the form of a rectangular plate of bone and wedge it in position to try and hold the lamina in its new, more open shape. This is generally effective but because it is not a firm arrangement, it can lead to some slippage and recurrent narrowing of the spinal canal. It also involves making a separate wound in the area of the *Illium* and taking a bone graft. Another technique uses a surgical implant device. In percutaneus laser disc decompression (PLDD), a thin needle is inserted into the herniated disc at a forty-five degree angle, using Novocain for local anesthesia and X-ray guidance. An optical fiber is then inserted into the needle an laser beam is sent through the fiber, vaporizing a tiny portion of the disc nucleus. This creates a partial vacuum, which draws the herniation away from the nerve root, thereby relieving pain. In this area of needle placement there are no vital structures that are dangerous to the vertebral column, as exiting nerves or blood vessels. PLDD is different from open lumbar disc surgery because there is no damage to the back muscle, no bone removal and no large skin incision. Lasers were initially considered ideal for spine surgery because lasers ablate or vaporize tissue with heat, which also acts to cauterize and seal the small blood vessels in the tissue.

U.S patent No. 5,865,833 discloses apparatus for laser treatment for treating the tissue of a human body such as herniated lumbar intervertebral disc by irradiating it with *laser* light for vaporizing it.

Unfortunately, lasers are both expensive and somewhat tedious to use in these procedures. Another disadvantage with lasers is the difficulty in judging the depth of tissue ablation. Since the surgeon generally points and shoots the *laser* without contacting the tissue, he or she does not receive any tactile feedback to judge how deeply the laser is cutting. Because healthy tissue, bones, ligaments and spinal nerves often lie within close proximity of the spinal disc, it is essential to maintain a minimum depth of tissue damage, which cannot always be ensured with a laser.

Electrosurgical spine surgery is an alternative to PLDD that is also aimed for nucleus pulposus removal by energy. U.S patent No. 6,283,961 discloses herniated disc treatment within a patient's spine by applying sufficient high frequency electrical energy through one or more suitable electrodes to the disc tissue to reduce the volume of the disc, thereby relieving pressure on a spinal nerve. The high frequency energy may be sufficient to ablate a portion of the nucleus pulposus within the annulus. The electrode terminal is advanced into the annulus and sufficient high frequency voltage is applied to contract or shrink the collagen fibers within the nucleus pulposus. This causes the nucleus pulposus to shrink and withdraw from its impingement on the spinal nerve.

A complementary procedure to Nucleus pulposus removal is regeneration of the intervertebral disc space using an implant or an injectable material. Currently used injectable spacers are allograft-based.

U.S patent No. 6,283,966 discloses instruments and methods for positioning a spinal implant within an *intervertebral* disc space between adjacent vertebrae.

U.S patent No. 6,258,125 discloses a method for regaining intervertebral space using an intervertebral allograft spacer.

U.S patent No. 6,240,926 discloses a method for regaining intervertebral space using hybrid materials composed of a biodegradable synthetic material such as bioactive glass or polymer foam and isolated intervertebral discs cells. Document WO01/76654 discloses a fluid matrix comprising cross-linked remodelable collagen from a donor vertebrate animal, useful for regenerating hydrodynamic function in damaged intervertebral disc in vivo.

Deficiency of nucleus pulposus caused by degenerative changes or by surgical procedures of spine decompression may create secondary deterioration inside and around the disc.

The nucleus pulposus is sited within a chamber, the walls of which are made of the annulus fibrosus and the vertebral plates. The material, of which the nucleus pulposus is made, inhibits inflammation and vascular and neural proliferation within that chamber. Deficiency of nucleus pulposus material enhances freedom from that inhibition which may result in inflammation, and vascular and neural proliferation. This reaction may be further enhanced by macro-movements of the walls of the empty chamber.

Additionally, Lack of mechanical stabilization due to shrinking of the volume of the nucleus pulposus may result in gross-movements of the annulus fibrosus and eventually to the development of tears or fissures in the annulus fibrosus. Narrowing of the inter-vertebral space creates non-anatomical loads of the vertebral facet joints and may eventually lead to osteo-arthritic changes in those joints.

### SUMMARY OF THE INVENTION

There is therefore provided , in accordance with the subject-mater of claim 1, the use of an injectable fluid including collagen cross-linked with a reducing sugar for treatment of degenerate disc disease of a vertebral column.

The use includes pressure injecting into at least one intervertebral disc of the mammal a volume of an injectable fluid including collagen cross-linked with a reducing sugar to reach a selected pressure level within the disc.

There is further provided, in accordance with an embodiment of the present invention, therapeutically injecting a fluid containing cross-linked collagen into a mammalian intervertebral disc. The use includes injecting the fluid into the internal space of the disc using an injection pressure sufficient to elevate the intra-discal pressure to a selected pressure level.

There is further provided, in accordance with an embodiment of the present invention, an injectable preparation for injecting into an intervertebral disc for inducing fibrocartilage formation in vivo. The preparation may include an injectable fluid containing particles of collagen cross-linked with a reducing sugar.

There is further provided, in accordance with an embodiment of the present invention, a medicament for increasing the height of a patient having a hydrodynamic disc dysfunction in at least one intervertebral disc. The use includes injecting into at least one disc of the patient a volume of an injectable fluid including collagen cross-linked with a reducing sugar to increase the distance between the two vertebrae attached to the disc.

There is further provided, in accordance with an embodiment of the present invention, a medicament for increasing the height of a patient having a hydrodynamic disc dysfunction in at least one intervertebral disc. The use includes pressure injecting into at least one disc a volume of an injectable fluid including collagen cross-linked with a reducing sugar to reach a selected intra-discal pressure level within the injected disc(s).

There is further provided, in accordance with an embodiment of the present invention, a medicament for relieving back pain resulting from degenerative disc disease in a patient. The use includes injecting into at least one intervertebral disc of the patient a volume of an injectable preparation including a biocompatible biodurable material to increase the distance between the two vertebrae attached to the disc(s).

There is further provided, in accordance with an embodiment of the present invention, a medicament for at least partially restoring or preserving at least one mechanical property of at least one degenerative disc in a patient. The use includes injecting into at least one intervertebral disc of the patient a volume of an injectable preparation including a biocompatible biodurable material to increase the distance between the two vertebrae attached to the at least one disc.

There is further provided, in accordance with an embodiment of the present invention, a medicament for inducing at least a partial regeneration of the nucleus pulposus, or development of cartilaginous tissue or fibrocartilaginous tissue or dense fibrous tissues in a degenerative mammalian intervertebral disc. The method includes injecting into the disc an injectable preparation including collagen cross-linked with a reducing sugar.

### BRIEF DESCRIPTION OF THE DRAWINGS

The invention is herein described, by way of example only, with reference to the accompanying drawings, in which like components are designated by like reference numerals, wherein:

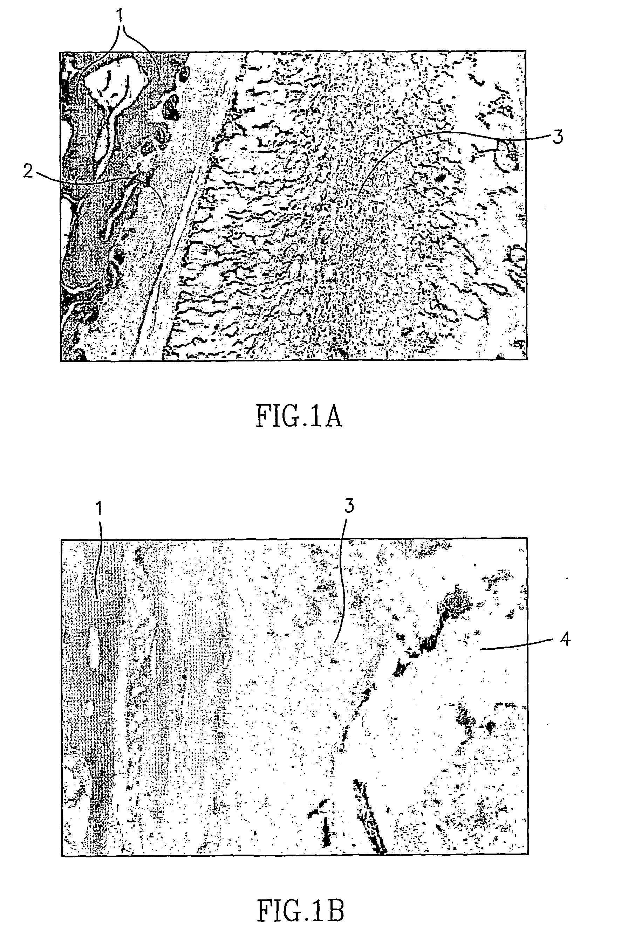

Figs. 1A-1G are photomicrographs representing histological sections taken from pig intervertebral discs obtained in EXPERIMENT 1, including control discs, sham-operated discs and discs injected with ribose cross-linked collagen, in accordance with an embodiment of the methods of the present invention;

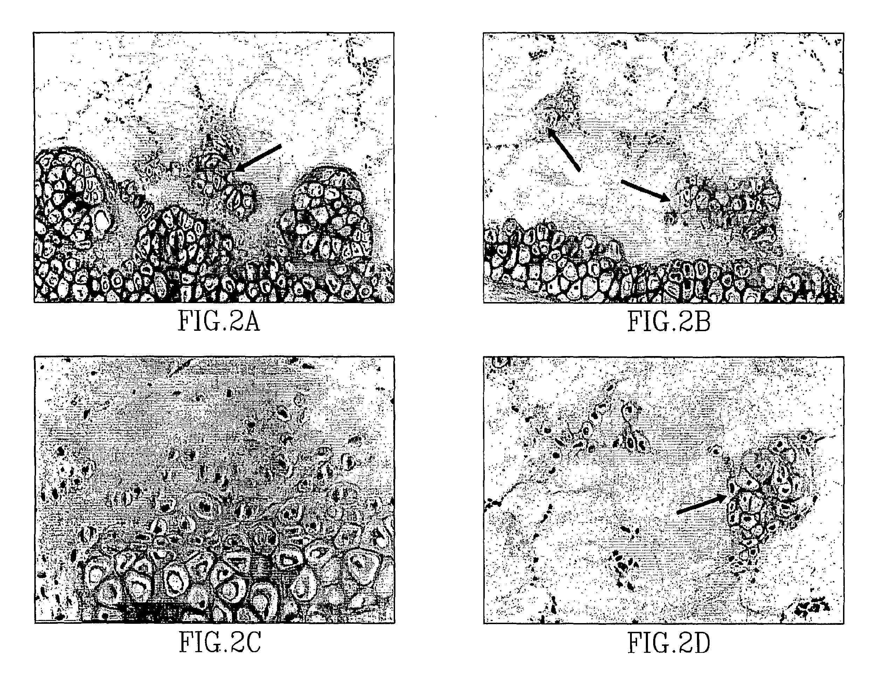

Figs. 2A-2D are photomicrographs illustrating the formation of cartilage tissue between particles of cross-linked collagen injected into the superficial part of rabbit ear cartilage;

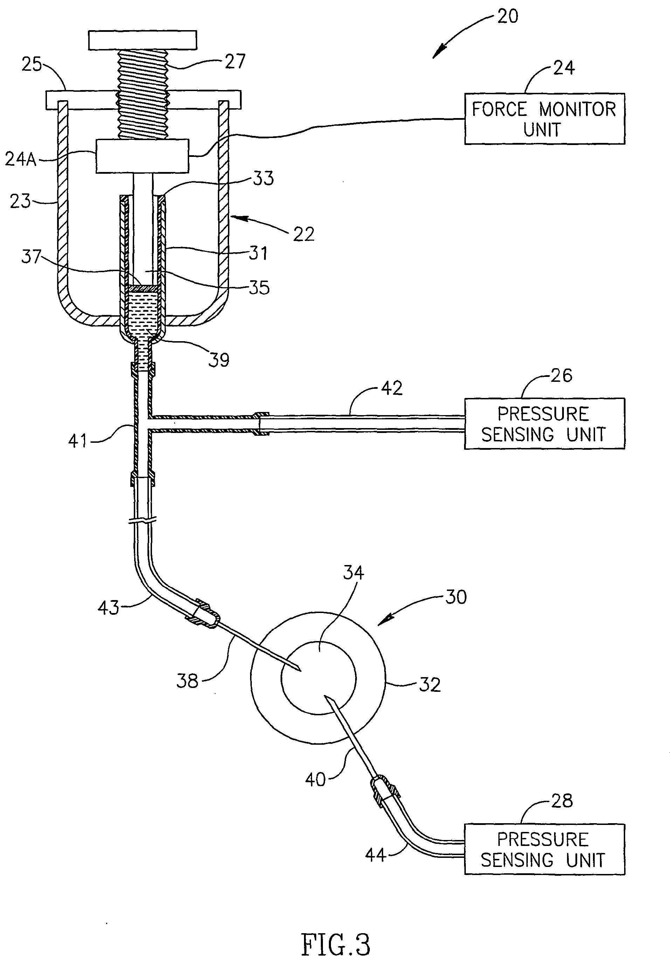

Fig. 3 is a schematic part cross-sectional diagram illustrating a system used for intra-discal injecting of ribose cross-linked collagen and for monitoring the force used for injection and the pressure at the injector outlet and in the intra-discal space, in accordance with an embodiment of the present invention;

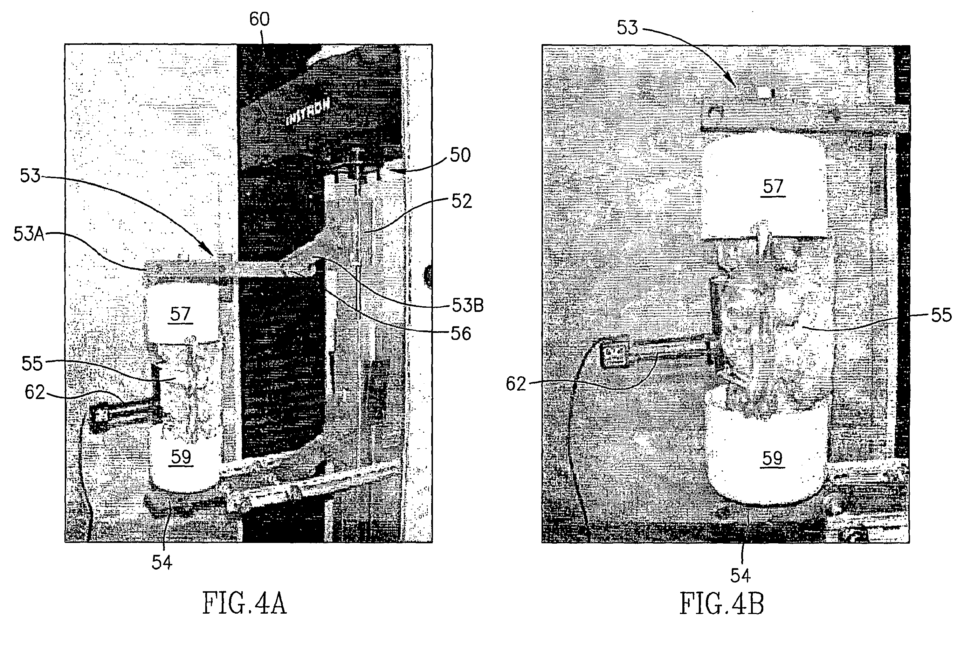

Figs. 4A-4D are photographs illustrating various configurations of the modified INSTRON device used in performing the biomechanical measurements including extension and flexion, and torsion experiments on pig spinal column segments from control animals and from animals having intervertebral discs injected with ribose cross-linked collagen, in accordance with an embodiment of the methods of the present invention;

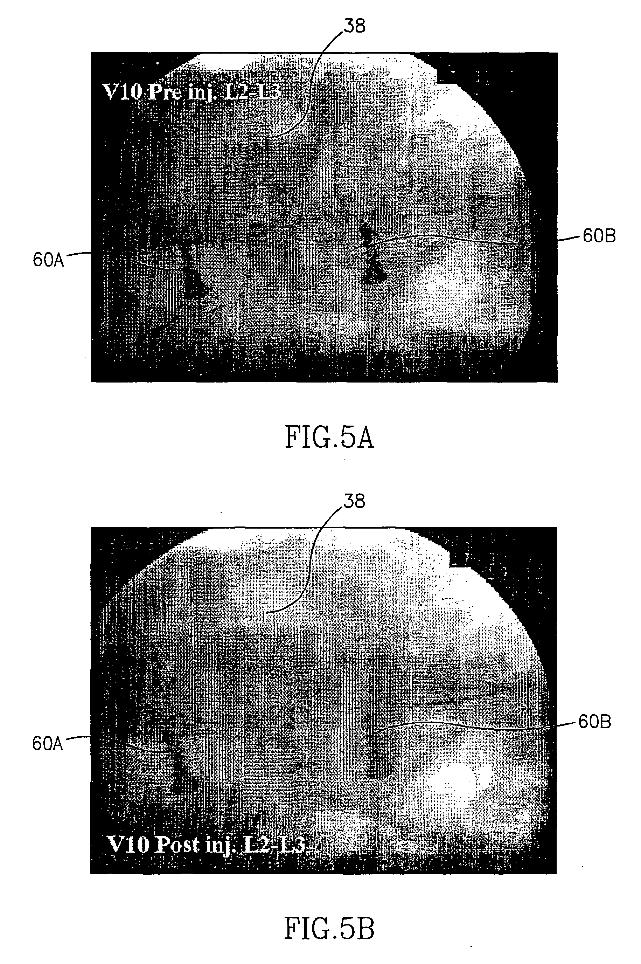

Figs. 5A-5D are X-ray images of parts of a pig spinal vertebral column illustrating the intervertebral spacing prior to and after intra-discal injection of ribose cross-linked collagen, in accordance with an embodiment of the methods of the present invention; and

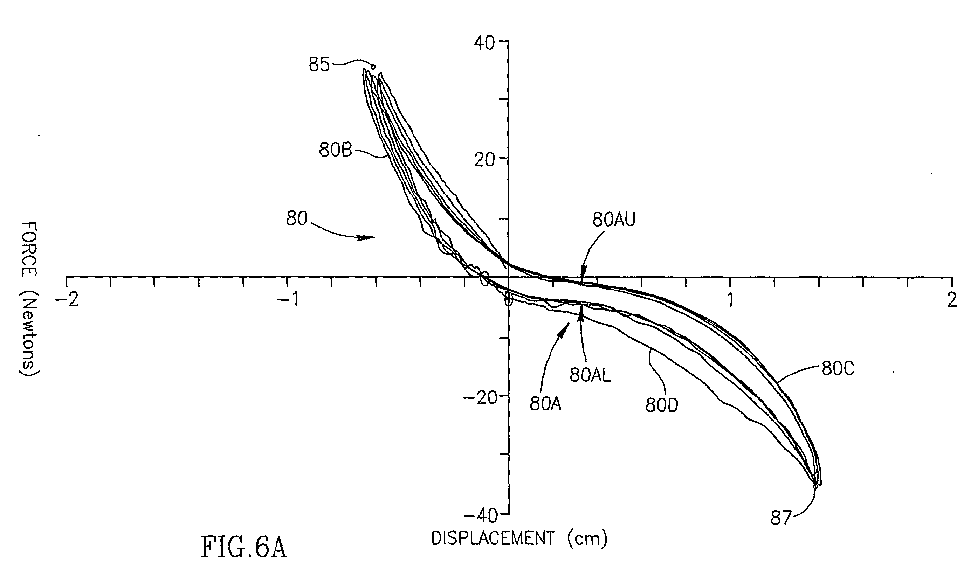

Figs. 6A-6F are schematic graphs illustrating curves representing examples of results of extension, flexion and torsion experiments performed on dissected pig spinal column segments from control and experimentally treated pigs having intervertebral discs injected with ribose cross-linked collagen, in accordance with an embodiment of the methods of the present invention.

### DETAILED DESCRIPTION OF THE INVENTION

### Notation Used Throughout

The following notation is used throughout this document.

| **Term** | **Definition** |
|---|---|
| IVD | Intervertebral disc disease |
| PLLD | Percutaneous laser disc decompression |
| PDN | Prosthetic disc nucleus |
| PLIF | posterior lumbar interbody fusion |
| VEGF | vascular endothelial growth factor |
| DDD | Degenerative disc disease |

The present invention discloses treating intervertebral discs having a nucleus pulposus deficiency or other types or discopathies or degeneracy by using a medicament comprising a long acting biocompatible crossed-linked collagen, applied into the intervertebral disc or another suitable injectable material that is biocompatible, and preferably also bio-durable. Preferably, the injected material or filler has water retaining capabilities. The cross-linked collagen may be introduced into the discal space which was occupied by the deficient nucleus pulposus. The cross-linked collagen or the other injectable filler material may mechanically stabilize the disc and the adjacent vertebrae.

In addition, the introduced cross-linked collagen may induce or promote the formation of fibrocartilage-like tissue within the chamber that may comprise fibrillar cross-linked collagen particles embedded within a cartilage-like or fibrous-like tissue, which may contribute to long term stabilization of biomechanical properties of the treated intervertebral to prevent discal deterioration processes.

The cross-linked collagen preparation may be introduced an the emptied nucleus pulposus space or into the intra-discal space of a degenerative intervertebral disc (without removing the nuclus pulposus). The procedure may be applied on painful intact annulus fibrosus discs using injectable cross-linked collagen preparations. Alternatively or additionally, the procedure may be performed on herniated discs having a damaged or fissured annulus fibrosus by utilizing compressed dry cross-linked collagen preparation.

Additionally, the injection procedure of the present invention may be used together with or in combination with other known therapeutic methods, including but not limited to, sealing of fissures or cracks in the annulus fibrosus using laser treatment or other types of heat treatments, as is known in the art.

### Performing the procedure on non-herniated degenerated discs

In accordance with one preferred embodiment of the present invention, the cross-linked collagen preparation is injected into non-herniated degenerated intervertebral discs. The degenerated disc may be penetrated by a hollow needle or by another suitable hollow injecting device or member having a sufficiently small diameter to minimize damage to the annulus fibrosus. The material within the nucleus pulposus is washed out and an injectable preparation comprising biocompatible cross-linked collagen is injected into the washed or partially washed space. The amount of collagen based preparation which is injected should be sufficient to at least partially restore disc shape, disc size, the spacing between the vertebrae, and other biomechanical properties and function of the treated disc and of the spinal vertebral column.

The injected material may act as a space maintainer and as a pain reducer. While the procedure may be performed by itself on painful discs, it may also be applied as a complementary procedure to disc suction or intradisc radio frequency wave warming procedures. The outer part of annulus fibrosus, must be intact since ruptures or fissures traversing the annulus fibrosus may allow leakage or escape of the injected material which may be an injectable liquid suspension.

Thus, the intervertebral disc repair method of the present invention may comprise removal by washing out or other suitable methods of some or all of the contents of the nucleus pulposus followed by introducing into the region previously occupied by the nucleus pulposus an injectable preparation comprising cross-linked collagen with or without additional materials or cells.

A feasibility pre-clinical study was performed. Two types of degenerative models were established. A first degenerative model used nucleus pulposus needle-washing, and a second degenerative model involved nucleus pulposus removal via an annulus stab as is known in the art. These models are described in detail hereinafter in Experiments 1 and 3, respectively.

### EXPERIMEMT 1

Pigs *(Sus domesticus)* weighting 50-60 kilograms were anesthetized. General anesthesia is established by a veterinary using fluoten, and an inter-tracheael tube. An anterior approach (specifically, a right retro pleural approach) to the thoracic vertebrae, was used. A sterile opening is made between the 6^{th} and the 7^{th} ribs. The ribs are spread apart and the lung with its wrappings is moved for obtaining a direct approach to the thoracic vertebrae. When the anterior approach is completed a retractor is applied and the breathing volume is decreased. A 22 gauge needle (22G) was introduced through the opening into the T6-T9 vertebrae region, and inserted through the annulus fibrosus of a intervertebral disc (selected from the discs of vertebrae T6, T7 or T8) into to the nucleus pulposus of the disc.

The insertion was performed at the right side of the disc. A high-pressure injector was connected to the 22G needle. Another 18 gauge needle (18G) was inserted into the nucleus pulposus of the same disc through the annulus fibrosus of the disc using a frontal disc penetration site. The 18G needle served as a draining outlet for washing out the contents of the nucleus pulposus. 10-20 milliliters of sterile saline was injected through the 22G needle at a pressure of about 10 atmospheres until the saline coming out of the 18G drainage needle was visually observed to be clear indicating that most of the material of the nucleus pulposus has been removed. A quantity of 0.5-2.0 milliliters of an injectable ribose crossed linked porcine collagen preparation was injected under pressure through the 22G needle until non-diluted collagen was visually observed to come out of the 18G needle used for drainage.

After the injection of the initial quantity of collagen is completed, the 18G drainage needle was withdrawn from the annulus fibrosus and another quantity of approximately 0.1-0.2 milliliters injectable cross-linked collagen is injected through the 22G needle until the collagen leaks out of the eyelet created by the 18G needle removal. The 22G needle is then withdrawn from the disc and a non-absorbable nylon stitch was made next to the treated vertebra for future identification of the injected discs. Suitable cancellous screws were also inserted into the vertebrae, for identification and intervertebral spacing measurements, as disclosed in detail hereinafter.

In each pig, the collagen injection is performed only on two out of the three vertebrae. The nucleus pulposus of the third vertebra was washed out by injection of 10-20 milliliters of sterile saline as disclosed hereinabove but no collagen injection was performed. This third vertebra was used as a control.

The thorax opening was stitched, the air was evacuated from the chest, and the pig was awakened.

### Results of EXPERIMENT 1

### Macroscopic examination

At sacrifice, the injected (experimental) discs were examined for ribose cross-linked collagen extrusion from the disc and any macroscopic pathological changes as abscess formation, inflammation and integrity of the annulus fibrosus.

### Histological Examination

Four discs were removed from each pig: two experimental and one sham-operated disc, which were identified by the stitches or the screw (placed at the time of operating on the animal), and one virgin (untouched) disc (basic control) from above or below the three treated discs (i.e. T5-T6 or T9-T10). The specimens were trimmed by transversally sectioning the spine to include the disc and the adjacent end plates. The specimens were fixed in buffered formalin and demineralized in formic acid. Histological sections were prepared along a coronary plane and stained with Hematoxylin and Eosin stain (H&E) as is known in the art, or with an Alcian Blue stain (pH = 1.0) as is known in the art, for visualizing proteoglycans.

The following points (a-c) were determined and recorded at the histological level with respect to tissue responses:
a. Inflammatory response.
b. Ribose cross-linked collagen integration within the disc tissue.
c. Ribose cross-linked collagen bio-durability.
TABLE 1 below lists the details of the animals used in the histology examination experiments.

**TABLE 1**

| **Pig No.** | **Duration After Operation** | **Comments** |
|---|---|---|
| **V5** | 1 month | Histopathological evaluation completed. |
| **V4** | 3 months | Histopathological evaluation completed. |
| **V7** | 3 months | Histopathological evaluation completed. |
| **V1** | 6 months | Histopathological evaluation completed. |
| **V8** | Not sacrificed yet | Pig reserved for follow-up at 12 months post operation. |

A total of nine pigs participated in the study (labeled as pigs V1-V9). Histological evaluation was obtained from 4 out of 5 pigs which were sacrificed at 1, 3 and 6 months post operation (pigs V1, V4, V5, and V7). An additional pig (pig V8) was reserved for sacrificing at 12 months after the operation (histology results are not available yet for this animal). The four remaining pigs (pigs V2, V3, V6 and V9) were rejected due to infection or death during the operation that was not related to the administration of ribose cross-linked collagen.

The histology results are summarized in TABLE 2 below (and also presented in Figs. 1A-1G of the drawing figures).

**TABLE 2**

| Fig. No. and (Pig. No.) | Disc Type | Months Post-operatively | Staining method | magnification |
|---|---|---|---|---|
| 1A (Pig V4) | Virgin thoracic | _ | H & E | X 40 |
| 1 B (Pig V5) | Sham-operated | 1 | H & E | X 40 |
| 1C (Pig V5) | Injected with collagen | 1 | H & E | X 200 |
| 1 D (Pig. V5) | Injected with collagen | 1 | Alcian Blue | X 200 |
| 1 E (Pig V4) | Sham-operated | 3 | H & E | X 40 |
| 1 F (Pig V4 ) | Injected with collagen | 3 | H & E | X 40 |
| 1G (Pig V1) | Injected with collagen | 6 | H & E | X 40 |

Reference is now made to Figs. 1A-1G which are photomicrographs representing histological sections taken from pig intervertebral discs obtained in EXPERIMENT 1, including control discs, sham-operated discs and discs injected with ribose cross-linked collagen, in accordance with an embodiment of the methods of the present invention.
Fig. 1A illustrates an H&E stained histology cross section (at x40 magnification) from a virgin (non-operated) disc (obtained from pig V4). Regions of bone 1, cartilage 2, and the nucleus pulposus 3 are visualized.
Fig. 1B illustrates an H&E stained histology cross section from a control sham-operated disc harvested 1 month post -operatively (obtained from pig V1). The photo micrograph reveals the formation of an unfilled cavity 4 containing some debris.

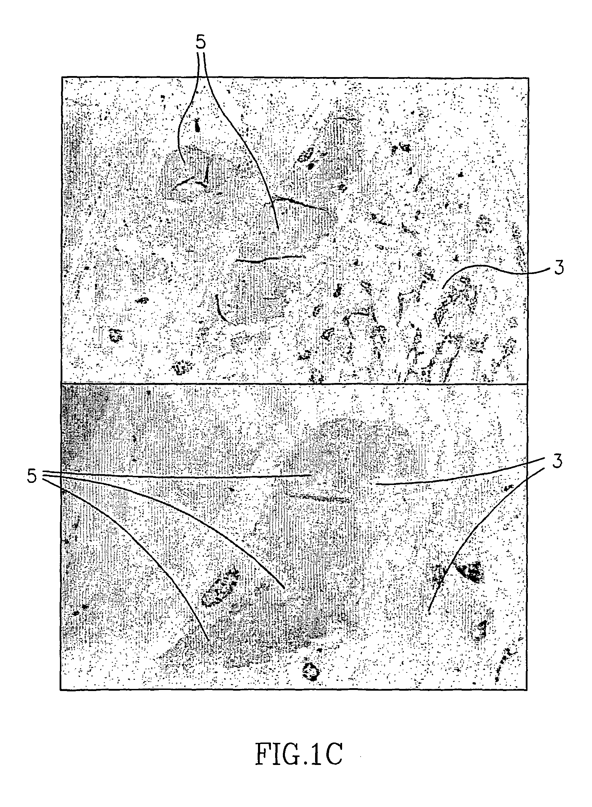

Fig. 1C illustrates two different H&E stained histology cross section photomicrographs (the top photomicrograph at x40 magnification, and the bottom photomicrograph at x200 magnification) taken from a disc injected with ribose cross-linked collagen and harvested 1 month post-operatively (obtained from pig V5). The photomicrographs reveals that the injected ribose cross-linked collagen 5 is surrounded by hyaline cellular tissue. The intimate contact between the ribose cross-linked collagen 5 and the hyaline matrix of the nucleus pulposus 3 may be seen.

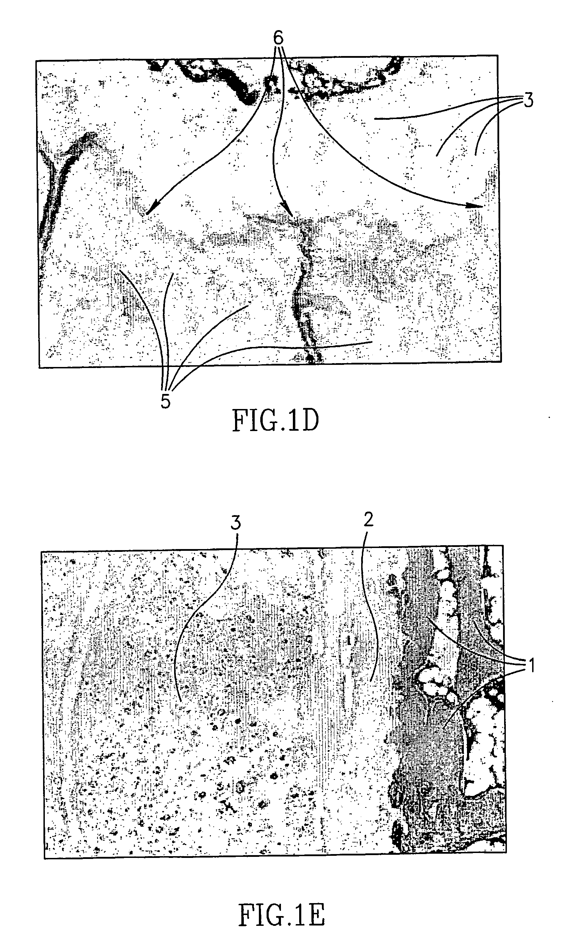

Fig. 1D illustrates an Alcian Blue stained histology cross section (at x200 magnification) from a disc injected with ribose cross-linked collagen and harvested 1 month post-operatively. The border 6 between the native tissues of the nucleus pulposus 3 and the injected Ribose cross-linked collagen 5 was basophilically stained, evidence to the absorption or in vivo deposition of proteoglycans onto the injected Ribose cross-linked collagen 5.
   No inflammatory reactions (no inflammatory cells penetration), no granulation tissue, and no proliferation of blood vessels were seen in any of the specimens of animals harvested 1 month post-operatively.
Fig. 1E illustrates an H&E stained histology cross section (at x40 magnification) from a control sham-operated disc harvested 3 months post - operatively (obtained from pig V4). The empty space or cavity observed at 1 month could not be identified in this three month post operative section and the cavity was filled by high cellular hyaline tissue of the nucleus pulposus 3.

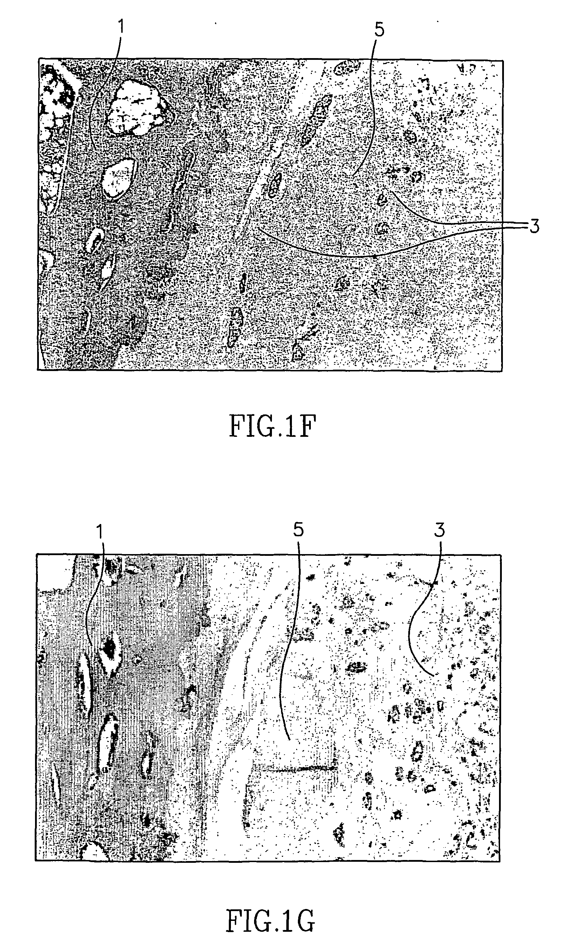

Fig. 1F illustrates an H&E stained histology cross section photomicrograph (at x40 magnification), taken from a disc injected with ribose cross-linked collagen and harvested 3 month post-operatively (obtained from pig V4). An intimate interaction of the ribose cross-linked collagen 5 with the adjacent hyaline tissue of the nucleus pulposus 3 is observed. No inflammatory reaction (no inflammatory cells penetration), no granulation tissue and no proliferation of blood vessels were seen in any of the specimens harvested three months post-operatively.
Fig. 1G illustrates an H&E stained histology cross section photomicrograph (at x40 magnification), taken from a disc injected with ribose cross-linked collagen and harvested 6 month post-operatively (obtained from pig V1). Similar to the section illustrated in Fig. 1F, an intimate interaction of the ribose cross-linked collagen 5 with the adjacent hyaline tissue of the nucleus pulposus 3 is observed. No inflammatory reaction was observed. Cells did not invade the ribose cross-linked collagen 5 during the 6 months experimental period. No granulation tissue and no proliferation of blood vessels were seen. Even though no quantitative measurements were performed, no signs of ribose cross-linked collagen degradation were observed during this period.

To summarize the histology results of EXPERIMENT 1, excellent tissue response between the disc tissues and ribose cross-linked collagen was observed at all time points up to six months after disc injection. No inflammatory reaction occurred, as could be seen by the absence of inflammatory cells and the lack of proliferation of blood vessels. The histological sections, representing discs harvested 1, 3 and 6-months post injection demonstrate intimate interaction between the Ribose cross-linked collagen 5 and the remaining hyaline tissue of the nucleus pulposus 3 without the formation of a separating membrane or capsule characteristic to foreign body reactions. This adjacent affinity supports the notion that replacement (even partial) of the nucleus pulposus with Ribose cross-linked collagen is feasible. Proteoglycans were identified within the injected Ribose cross-linked collagen. The in vivo absorption or deposition of proteoglycans may contribute to the visco-elastic properties of Ribose cross-linked collagen. This feasibility study indicates that Ribose cross-linked collagen has the adequate biophysical properties to act as an excellent replacement material for the vertebral nucleus pulposus.

### EXPERIMENT 2

This experiment tested the induction of cartilage formation by porcine and bovine ribose cross-linked atelopeptide collagen. Injectable cross-linked bovine collagen and injectable cross-linked porcine collagen were tested as to their ability to induce cartilage formation in rabbit ear cartilage.

Injectable cross-linked bovine collagen and injectable cross-linked porcine collagen (100 microliters per injection site) were injected intra-cutaneousely into the ears of 20 New Zealand rabbits, using a 30 gauge (30G) needle. The needle penetrated the cartilage of the ear. Biopsies from the injected sites were obtained at one month post-injection and processed for histological examination.

All the injected sites for both porcine and bovine injectable cross-linked collagen could be physically identified at 1 month, 6 months and one year post-injection. No differences were observed in the tissue response to injectable cross-linked bovine collagen as compared to injectable cross-linked porcine collagen. Fibro-cartilage tissue was observed growing near and in the injected collagen based material. When cross-linked collagen was injected into and adjacent to the rabbit ear cartilage, chondroplasts were observed to populate the injected collagen matrix, forming cartilage structure in-between the cross-linked collagen particles. The general histological appearance was of a composite tissue consisting of cartilage reinforced with collagen particles. Some of these particles were also colonized by chondroblasts, and fibroblasts.

Reference is now made to Figs. 2A-2D which are photomicrographes illustrating the formation of cartilage tissue between particles of cross-linked collagen injected into the superficial part of rabbit ear cartilage as disclosed hereinabove. As can be seen in Figs. 2A-2D, chondroblasts migrated in between the injected cross-linked collagen material. In some sited indicated by the arrows, the condroblasts developed into islands of cartilage tissue. The biopsies of Figs. 2A-2D were obtained one month after injection.

The results illustrated in Figs. 2A-2D demonstrate the ability of the injected collagen preparations to promote the induction of cartilage and fibrocartillage formation in vivo. Therefore, when applied to intra-discal injection in humans, the therapeutic methods of the present invention may result in at least a partial regeneration of the nucleus pulposus, and/or development of cartilaginous or fibrocartilaginous tissues or dense fibrous tissues in the intra-discal space.

### EXPERIMENT 3

### Biomechanical examination

The experiment is designed to characterize the biomechanical features of degenerated intervertebral disc treated with ribose cross-linked collagen. These features are compared to the biomechanical features of healthy and non-treated degenerated discs. Macro assessments and histological examination of degenerated intervertebral disc treated with cross-linked collagen were performed.

The procedure to experimentally create a model of degenerative disc disease (DDD) in animals was described in several papers (Habtemariam et al., 1998; Kanerva et al., 1997). Briefly, pigs underwent anesthetization and a left retroperitoneal approach was used. The fibers of the *external oblique, internal oblique* and the *transversus abdominis* muscles were separated. The peritoneum was identified and separated from the muscles and the Psoas muscle was identified. Due to the pig anatomy a posterior dissection was performed and entry was done between the *Psoas* and the vertebrae and not through an anterior dissection, in front of the *Psoas* muscle, as in humans. The Psoas was separated form the transverse processes, a retractor was inserted and the lumbar vertebral column was exposed.

Using a lateral X-ray imaging, vertebrae L1-L4 were identified. The X-ray imaging was performed using a mobile C Arm, image intensifier Roentgen machine (Phillips, model V-300). After separation and gentle mobilization of the segmental blood vessels, cancellous A.O. screws (12 millimeter long, 4.0 millimeter outer thread diameter) were inserted, one screw into each vertebral body. The distance between the screws was measured using a metal caliper.

An approximately 1.5 centimeter stab incision was made with a No. 11 scalpel blade into the antero-lateral annulus fibrosus of L2-L3 and L3-L4 discs, parallel to the end plates, penetrating into the nucleus pulposus. Part of the nucleus was removed. The wound was closed in layers and the pig was awakened.

EXPERIMENTAL GROUP: This group included six pigs (pigs V10, V11, V12, V15, V16, and V17 of TABLE 3 below). At time zero (four months after the first operation), the pigs were anesthetized again and the operated degenerative discs were injected with 1.5 ± 0.2 milliliters of an injectable porcine ribose cross-linked collagen preparation (at a concentration of 35 milligram cross-linked collagen per milliliters of PBS). The injection was performed through a 22G spinal needle, using a pressure injection system capable of producing pressures of up to 10 atmospheres. The pressure injection system is described in detail hereinafter (See Fig. 3). The injection was done percutaneously using the image intensifier mobile Roentgen machine as disclosed hereinabove. The needle was inserted anterior and adjacent to the transverse processes of the vertebrae through the psoas muscle and the lateral side of the disc to the center of the disc while the animal was lying on its right side.

CONTROL GROUP: This group included five pigs that were operated to establish the DDD (pigs V13 ,V14, V18, V19, and V20 of TABLE 3 below). This group of animals was not injected with cross-linked collagen at zero time and served as a control.

SPINAL CANAL AND EPIDURAL INJECTION GROUP: Five pigs (pigs R10, R11, U3, U4, and U5 of TABLE 3 below) were tested to find out the reaction to an accidental leak of ribose cross-linked collagen into the spinal canal. In pig R10, under posterior open approach, 1.0 milliliter of ribose cross-linked collagen was injected into the intra-dural (spinal) space of the spinal canal at about L2-L3 and L3-L4 levels of one pig. In the remaining four pigs an epidural injection of approximately 1.0 milliliter ribose cross-linked collagen was performed. This was done in order to mimic the situation of a defect in the posterior wall of the annulus fibrosus leading to the extrusion of the material into the spinal canal. The animals were monitored for any development of neurological signs such as limping and incontinence. Six months after injection the animals will be sacrificed and the area of injection histologically examined.

Reference is now made to Fig. 3 which is a schematic part cross-sectional diagram illustrating the system used for intra-discal injecting of ribose cross-linked collagen and for monitoring the injection force and the pressure at the injector outlet and in the intra-discal space, in accordance with an embodiment of the present invention.

The system 20 includes an injecting device 22 for injecting the cross-linked collagen, a force monitor unit 24 and a force transducer 24A for measuring force, and two pressure sensing units 26 and 28.

The intervertebral disc 30 is schematically illustrated in a transversal cross sectional view to show the annulus fibrosus 32 and the nucleus pulposus 34. An injecting needle 38 and a pressure sensing needle 40 are shown in their positions after being inserted through the annulus fibrosus such that their open needle tips are disposed within the nucleus pulposus 34 (or the degenerated nucleus pulposus in DDD model animals) in the intra-discal space of the intervertebral disc 30.

The injecting device 22 includes a holding frame 23. The holding frame 23 is made from autoclavable surgical steel and is attached to a stainless steel back-plate 25. A threaded hole is formed in the back-plate 25, and a forcing screw 27 is movably disposed within the threaded hole. The holding frame 23 is also attached to a metal sleeve 31.

A standard 5 milliliter plastic syringe 33 may be placed within the sleeve 31. A stainless steel rod 35 attached to a rubber piston 37 may be inserted into the syringe and functions as a plunger for pushing the ribose cross-linked collagen preparation 39 disposed within the syringe 33. A high-pressure T-junction 41 is connected to the (Luer type) lock of the syringe 33. One arm of the T-junction 41 is connected through a reinforced high pressure flexible tube 42 to the pressure sensing unit 26 for measuring the pressure at the outlet of the syringe 33. The second arm of the T-junction 41 is connected through a reinforced high pressure flexible tube 43 to a standard stainless steel injection needle. A 22 Gauge (22G) needle was used in the experiments. It is, however, noted that other types and gauges of needles may also be used and that the internal and external diameter of the injection needle 38 may be changed depending ,inter alia, on the viscosity and concentration of the injectable cross-linked collagen preparation used, on the method of inserting the injection needle 38 into the interverteral disc 30, and on other practical considerations.

In the experiments of the present invention, the pressure sensing needle 40 was a standard stainless steel Luer lock type, 18 gauge (18G) needle. The pressure sensing needle 40 was connected through a reinforced high pressure flexible tube 44 to the pressure sensing unit 28 for measuring the intra-discal pressure (the pressure within the disc), before, during and after the injection of the ribose cross-linked collagen preparation 39.

The pressure sensing units 26 and 28 used in the experiments were model INDEFLATOR PLUS 20^{™} inflation devices including analog pressure gauge, commercially available from Guidant Advanced Cardiovascular systems, Inc., CA, USA. These devices have a measuring range of up to 20 atmospheres. Other suitable types of pressure sensing devices may also be used.

In the experiments, the force transducer 24A was attached between the forcing screw 27 and the end of the rod 35 for measuring the force acting on the rod 35. The force monitor unit 24 and the force transducer 24A were parts of force gauge unit commercially available as model FG-100kg force gauge from LUTRON Electronic Enterprise Co. Ltd. Taipei, Taiwan. The force gauge had a range of up to 100 kilograms of force with a nominal resolution of 50 grams of force.

In the experiments, the syringe 33 was loaded with ribose cross-linked collagen preparation containing ribose cross-linked porcine collagen at a concentration of 35 milligrams per milliliter of phosphate buffered saline (PBS). The forcing screw 27 was screwed in to exert pressure on the end of the rod 35 and to fill the T-junction 41, the tube 42 and the injection needle 38 with the injectable collagen preparation. Air was expelled by using PBS from the T-junction 41, the tube 42 and the needle 38 prior to insertion of the injecting needle 38 to enable pressure measurement by the pressure sensing unit 26 and to avoid the injection of air into the intra-discal space.

After the needles 38 and 40 were inserted into the disc 30 as disclosed in detail herein, the position of the end of the rubber piston 37 was recorded, and the forcing screw 27 was used to exert an increasing force on the end of the rod 35. The screw was advanced until a relatively stable pressure level of approximately 6-11 atmospheres was developed at the syringe outlet monitored by the pressure sensing unit 26. The pressure at the outlet of the syringe 33 and at the space within the intervertebral disc 30 was visually monitored on the pressure sensing units 26 and 28, respectively, and manually recorded, before during and after the injection of the cross-linked collagen preparation. The force on the end of the rod 35 was visually read from the display of the force monitor unit 24 and manually recorded.

After the injection was completed (approximately 1-2 minutes after pressure stabilized at the 6-11 atmosphere level, the position of the end of the rubber piston 37 was again recorded to determine the amount of the cross linked collagen preparation which was injected into the disc 30.

Preferably, it may be desired to therapeutically inject ribose cross-linked collagen preparations having as high a concentration of cross-linked collagen as practically possible into the intra-discal space to increase the restoration of intervertebral space and mechanical properties as close as possible to normal values, and to ensure a long lasting therapeutic effect (in view of the slow biodegradation of the injected cross-collagen material *in vivo* by endogenous collagenases).

Practically, however, the amount of collagen which may be injected may be limited by the highest concentration of ribose cross-linked collagen which may be injected through the needle used without using excessively high pressure levels and without clogging the injecting needle 38 by collagen particles. Additionally, while one would like to decrease the gauge of the needle 38 in order to minimize the size of the puncture in the annulus fibrosus, the decreasing the needle diameter (increasing the needle gauge) may be limited, inter alia, by the ability to force the cross linked collagen to flow through the needle and by the mechanical properties of the needle. For example, the use of very thin needles may be practically limited by the ability to controllably direct a thin needle through the tissues and to insert the needle through the annulus without bending or flexing of the needle.

It is noted that in various preliminary tests it was found that the injection system 20 may be successfully used to inject into the intra-discal space of pigs ribose cross-linked collagen preparations having concentrations comprised between 35 and 55 milligrams of cross-linked collagen per milliliter through needles having a gauge in the range of 22G - 31 G without clogging of the needle, by using pressure levels at the outlet of the syringe 33 which did not exceed 10 atmospheres.

Thus, based on the known similarities between the structure, size, and mechanical properties of porcine and human intervertebral discs, it may be possible to inject the ribose cross-linked collagen preparations of the present in the concentration between 35 and 55 milligrams/milliliters into the intra-discal space of intervertebral discs of mammals (including, but not limited to, porcine, canine and human discs).

The preferred pressure range for intra-discal pressure (the pressure within the intervertebral space of the disc) may be within the range of approximately 0.5-10 atmospheres with the most preferred pressure range (as measured with the pressure sensing unit 28 during the injections) may be approximately 6-8 atmospheres.

Preferably, the intra-discal pressure should not exceed 10 atmospheres to avoid the possibility of disc rupture.

For porcine and human discs, the volume of ribose cross-linked collagen preparation which may be safely injected into the intra-discal space may be in the range of approximately 0.5 - 2.0 milliliters. The injectable volume may vary depending, inter alia, on the age and weight of the animal or patient, the size of the injected disc and position of the injected disc within the spinal column, and on the degree of intervertebral disc degeneration present. Other factors may, however also affect the injectable volume such as, for example, pathological changes in the mechanical parameters of the annulus fibrosus.

### Macro Assessments

Macro inspection of the discs and the facet joints for any sign of arthritis or other local reaction will be performed. In order to measure the amount of the disc inter-vertebral space, an autoclavable metal caliper, with sharp pointed tips, with controlled opening, was used. The distance between the cancellous screws inserted into the vertebrae was measured at the initiation of the DDD and at the time of sacrificing the animal. At the time of the operation, measurements are taken before and after cutting the annulus. At the time of sacrifice we operated the pig through the same antero-lateral approach while the animal was laid on its right side as in the first operation and measurements are taken in this position.

The tips of the caliper were put in the holes that were in the heads of the screws and the caliper was gently closed until the tips grabbed lightly the inner walls of the holes in the head of the screw. The caliper was then removed, reinserted and re-adjusted a few times until it was evident that there was no change between the measurements due to the elastic effect of the caliper metal arms. A mark on a sheet of paper was then made by gently pressing the tips of the caliper into a paper. The distance between the marks was measured using a caliber meter with accuracy of 0.01 millimeters.

By comparing the measurement results that were obtained during at the first operation to those obtained at sacrifice, the amount of change in the intervertebral distance is assessed. The measurements of the untouched L1-L2 disc served as a control and as a way to estimate the changes in the vertebrae and disc dimensions due to animal growth.

### Histological Examinations

### Histological Examinations was performed as described hereinabove for EXPERIMENT 1.

### Biomechanical examinations

The stiffness of the spinal column in flexion, extension and torsion, were measured using an Instron 4502 Automated Material Testing System (commercially available from Instron Corp., MA, USA) to which the specifically designed attachments were connected. An infrastructure for biomechanical measurements of inter-vertebral disc functionality and movement was established. These measurements were used for assessing the efficacy of injectable cross-linked collagen in treating degenerative disc diseases in the animal models.

After sacrifice, intact spinal column segments including vertebrae L1 to L5 were retrieved from the sacrificed pig. The harvested segments included all the vertebral bony parts, joints, discs and ligaments without the attached muscles and skin. The column segments were deep frozen at -20°C. Before testing, the column segments were defrosted (Gleizes et al., 1998 has demonstrated that there is no difference in biomechanical results between fresh and thawed frozen spinal columns) and prepared for examination by connecting the spinal column segment at its ends with rigid plastic cement to the testing device as disclosed in detail hereinafter.

### Measurement systems

In order to measure the effects of loading modes (bending and torsion) on segments of the vertebral column two separate systems were designed. Both systems were connected to the Instron 4502 machine. The INSTRON 4502 Automated Material Testing System is a dynamometer with a load cell commercially available from Instron Corp., MA, USA. Data of load and movements are synchronized and continually fed to a Personal computer (PC).

Reference is now made to Figs. 4A-4D which are photographs illustrating two configurations of the modified INSTRON Automated Material Testing System used in performing the biomechanical measurements including bending, flexing, and torsion experiments on pig spinal column segments from control animals and from animals having intervertebral discs injected with ribose cross-linked collagen, in accordance with an embodiment of the methods of the present invention.

Figs. 4A and 4B illustrate the positioning of the pig spinal column segment in the configuration of the INSTRON machine used for testing bending of the spinal column. Fig. 4B illustrates a part of Fig. 4A in detail.

The bending model system is based on previous bending measuring systems, described in the literature (Chiba M. et al., 1996; Marmelstein LE. et al., 1998). This system is mechanically connected to the INSTRON machine base as seen in Figs. 4A-4D. Two 145 millimeters long arms 53 and 54 are each connected to the INSTRON machine through a rotational hinge.

The upper arm 53 includes a first part 53A and a second part 53B. The second part 53B is rigidly and fixedly attached to a linear ball bearing 52. The linear ball bearing 52 is coupled to a load cell 50 which is attached to the INSTRON machine bridge 60. The first part 53A of the arm 53 is movably connected to the second part 53B by an axle 56 which functions as a hinge to allow the first part 53A to move up and down in the vertical direction only (in a plane including the longitudinal axis of the spinal column segment 55} such that the angle between the part 53A and the fixed part 53B may change.

The lower arm 54 is rigidly attached to the chassis of the INSTRON machine. The arms 53 and 54 may thus exert a force off center the longitudinal axis of the spinal column segment 55 (see Fig. 4A). The first part 53A of the arm 53 may rotate at an angle to the horizontal plane to allow bending of the spinal column segment 55.

The end vertebrae of the spinal column segment 55 are fixed into plastic cups 57 and 59 using 3 screws (not shown) and a hard polyethylene casting material as disclosed in detail hereinafter. A 10 millimeter diameter screw (not shown) was fixed to each of the plastic cups 57 and 59 in order to attach the spinal column segment 55 to the mechanical loading system. The vertebrae at both sides of the tested spinal column segment 55 are embedded within the polyethylene casting and thus are fixed to the central region of the cups 57 and 59, while the screw (not shown) is situated to be at the axis of the vertebra's movement (which is approximately at the center of the intervertebral discs of the spinal column segment 55).

The spinal column segment 55 is fixed by the 10 millimeter screws of the cups 57 and 59 at an angle of 90° to the arms 53 and 54 and is positioned ex-center to the INSTRON bridge 60. When the INSTRON bridge 60 descends, the arms 53 and 54 move one towards the other resulting in a moment, which bends the vertebral column of the spinal column segment 55 in one direction, towards the bridge 60.

When the INSTRON bridge 60 ascends, the arms 53 and 54 move one away from the other resulting in a moment which bends the vertebral column of the spinal column segment 55 in another direction, away from the bridge 60.

Two bending directions (flexion and extension) may be obtained. The applied force is measured directly by the load cell 50.

The movement measurement in the spinal column segment 55 is conducted using an extensiometer 62 (commercially available as catalogue number 2620-601, from Instron Corp., MA, USA). The arms of the extensiometer 62 are attached to the tested vertebrae (L2 and L4) in the movement direction. The upper arm of the extensiometer 62 is attached to vertebra L2 and the lower arm of the extensiometer 62 is attached to the vertebra L4. The acquired data is processed to generate a stress-strain curve representative of the force versus extension measurements (see the exemplary graphs of Figs.6A-6C hereinbelow).

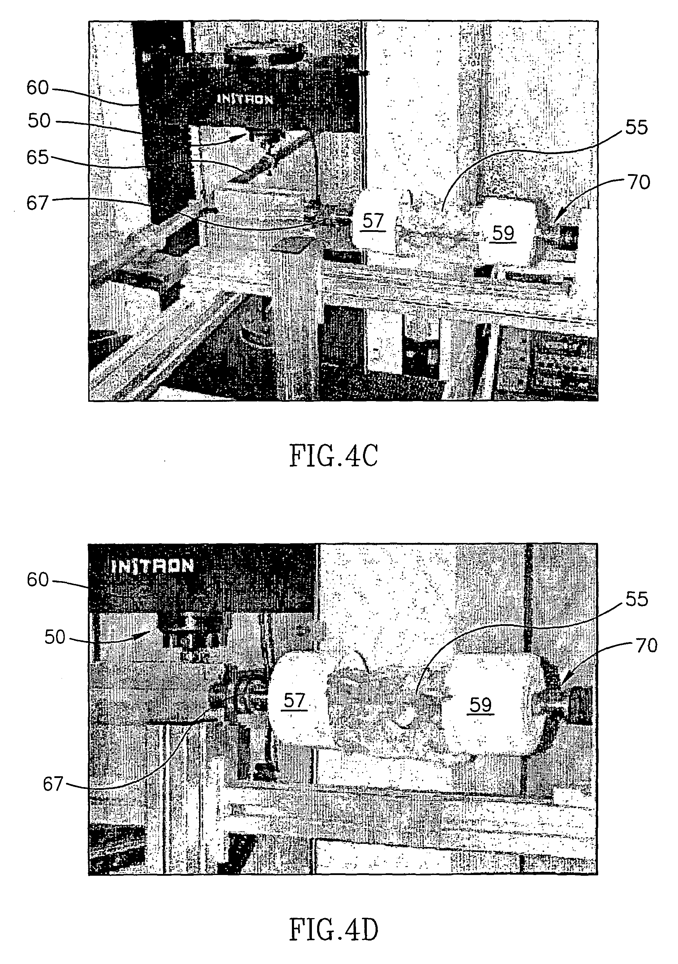

Figs. 4C and 4D illustrate the positioning of the pig spinal column segment in the configuration of the modified INSTRON machine used for testing torsion of the spinal column. Fig. 4D illustrates a part of Fig. 4C in detail.

The torsion model is based on an arm 65 connected to a rotatable axle 67, to which the vertebral column segment 55 is fixedly attached. Two screws (not shown) fix the ends of a vertebrae column segment to a polyethylene cement casting, cast within the plastic cups 57 and 59 as disclosed in detail hereinafter. The cup 59 may be fixedly attached to a fixed non-rotatable holder 70 having a chuck at its end, while the cup 57 may be connected to a chuck at the end of the rotatable axle 67. The movable arm 65 is rigidly attached to the axle 67. The INSTRON machine controls the movement of the arm 65 by being coupled to one end of the arm 65 through a load cell 50. The force applied by the machine creates a torsion moment on the rotatable axle 67, and on the vertebrae column segment 55 attached thereto. The moment may be calculated by multiplying the arm length by the applied force as measured directly by the load cell 50. The rotary movement, given in degrees, is calculated from the movement of the INSTRON bridge 60 and from the length of the arm 65. A stress-strain curve may then be computed based on the moment and the rotary movement data.

The applied forces in this study are within the range which does not damage the examined vertebral column segment (Chiba M, al., 1996; Marmelstein LE, 1998; Dick JC, 1997).

### Mechanical Examination Protocol

**Preparation method:** the lumbar vertebral column was thawed for 15 hours at room temperature. Muscles were removed, without damaging ligaments, capsules and discs. The end vertebrae were fixed into plastic cups using 3 screws and a hard polyethylene casting (commercially available as Por-A-Kast Mark 2, from Synair corporation, USA). A 10 millimeter diameter screw was fixed to each plastic cup in order to attach the column to the mechanical loading system. The vertebrae at both sides of the tested column are fixed to the central region of the cup, while the screw is situated to be at the axis of the vertebra's movement, which is at the center of the disc.

**Torsion Examination:** A prepared vertebral column was connected to the INSTRON machine as disclosed hereinabove and illustrated in Figs. 4C and 4D. The arm 65 is rigidly connected to the rotatable axle 67 having a chuck at its end. The chuck may be connected to the 10 millimeter screw attached to the plastic cup 57. Thus, the axle 67 may be rotated around the longitudinal axis of the lumbar spinal cord segment 55. One end of the arm 65 is connected to the INSTRON bridge 60 through a load cell 50. The length of the part of the arm 65 between the point of attachment of the arm 65 to the load cell 50 and the point of attachment of the arm 65 to the axle 67 is 250 millimeters. The other (opposite) end of the arm 65 is loaded with a load of 20 Newtons by a suitable weight (not shown in Fig. 4C) attached thereto (the weight is attached to the other end of the arm 65 at a distance of 250 millimeters from the point of attachment of the axle 67 to the arm 65).

At the beginning of each test, the bridge 60 is moved to position the arm 65 in a horizontal orientation. The lumbar spinal column segment 55 is positioned between the chuck of the axle 67 and the chuck of the fixed non-rotatable holder 70, and the 10 millimeter diameter screws attached to the cups 57 and 59 are rigidly locked within the chucks at the ends of axle 67 and the holder 70, respectively. The position of the bridge 60 at the time of locking is (arbitrarily) set as zero.

The INSTRON machine was programmed to impose a load of up to 40 Newton on the part of the arm 65 connected to load cell 50, and to move the bridge 60 down at a rate of 180 millimeters/minute (Fig 4C). The other opposite part of the arm 65 has a constant load of 20 Newton acting thereon due to the weight attached thereto. Therefore, the moment was 5 Newton x meter. Once the maximum load level of 40 Newtons is developed (as detected by the load cell 50), the INSTRON machine reverses the direction of movement of the bridge 60 and the moment is released at the same bridge movement rate (180 millimeters/minute) until a 0.125 Newton x meter moment is reached, at which point the direction of movement of the bridge 60 is again reversed.

Five consecutive cycles were conducted for each test and the data was synchronically acquired and pooled by the PC, in order to present the results in a Stress-Strain graph.

**Bending (flexion and extension) Examination**: A prepared vertebral column segment (such as, for example, the spinal column segment 55 of Fig. 4A) was connected to the INSTRON machine, such that the sagital plane of the column segment 55 is at the movement plane of the arms 53 and 54 (See configuration illustrated in Fig. 4B). Bending of the spinal column segment 55 corresponding to the flexion-extension movement of the column was thus obtained. The two arms of the extensiometer 62 were fixed to the vertebrae in the movement's direction. The test started with a load of 35 Newton. The Instron's bridge 60 was programmed to rise at a speed of 180 millimeters/minute, for generating traction forces, until a force of 35 Newton was obtained. The bridge 60 then descended again at the same speed until a compression load force of 35 Newton was reached. Five consecutive cycles were conducted and the data was synchronically acquired and pooled by the PC. The results were presented as stress-strain curves.

Since the arms' length is 145 millimeters, a 35 Newton loading force results in a 5 Newton x meter moment. However, since this is not a pure moment, but a bend, force values rather than moment values are presented.

Each of the tested spinal vertebral column segments underwent 5 cycles of loads and unloads in bending and in torsion at a maximum moment of 5 Newton x meters.

### Results of EXPERIMENT 3

Sixteen pigs were enrolled in the study. The allocation of the animals participating in EXPERIMENT 3 into the three groups mentioned above is shown in TABLE 3 bellow. Each of the experimental and control groups were subdivided into two subgroups A and B. Specimens obtained from subgroup A are designated for histological examination and those in subgroup B for biomechanical examination.

**TABLE 3**

| Time After collagen Injection | Histological Assessment (Subgroup A) | | Biomechanical Assessment (Subgroup B) | | Total No. of Pigs |
|---|---|---|---|---|---|
| - | Experimental Group | Control Group | Experimental Group | Control Group | |
| 4 days | V10 | - | - | - | 1 |
| 6 Months | V11-V12 | V13-V14 | V15-V17 | V18-V20 | 10 |
| 6 Months Epidural / Spinal | R10-R11 and U3-U5 | _ | _ | _ | 5 |

| | | | | | |
|---|---|---|---|---|---|
| V, R or U - represent the pig's designated group. Pig V10 was used to examine the feasibility of injecting cross-linked collagen under the image intensifier mobile Roentgen machine. Ribose cross-linked collagen was injected in 2 out of 4 of the degenerated discs, as disclosed hereinabove. | | | | | |

Reference is now made to Figs. 5A-5D which are X-ray images of parts of the spinal vertebral column of Pig V10 illustrating the intervertebral spacing prior to and after intra-discal injection of ribose cross-linked collagen, in accordance with an embodiment of the methods of the present invention.

In pig V10, 1.5 milliliters of porcine ribose cross-linked collagen preparation was injected into each of the intervertebral discs at the L2-L3 level and the L3-L4 level, using the injection system illustrated in Fig. 3. The pressure measured at the outlet of the syringe 38 (by the pressure sensing unit 26 of Fig. 3) at the end of the injections at the L2-L3 level and the L3-L4 level was approximately 9 atmospheres.

Figs. 5A and 5B represent the X-ray images in the region of Vertebrae L2 and L3 prior to and immediately after the injection of ribose cross-linked collagen into the intra-discal space, respectively. In Fig. 5A which was taken after the insertion of the injecting needle into the intervertebral disc space of the disc between vertebrae L2 and L3 but before the injection of the cross-linked collagen, part of the injecting needle 38 may be seen entering the intra-discal space from above. The screws 60A and 60B are the cancellous screws inserted into the L2 and L3 vertebrae, respectively, as disclosed in detail hereinabove.

The X-ray photograph illustrated in Fig 5B was taken immediately after the injection of approximately 1.5 milliliters of ribose cross-linked collagen having a concentration of 35 milliliters of porcine ribose cross-linked collagen into the L2-L3 intra-discal space, without moving the pig or the imaging machine during the entire injection and imaging procedure. The same screws 60A and 60B may be seen in Fig. 5B.

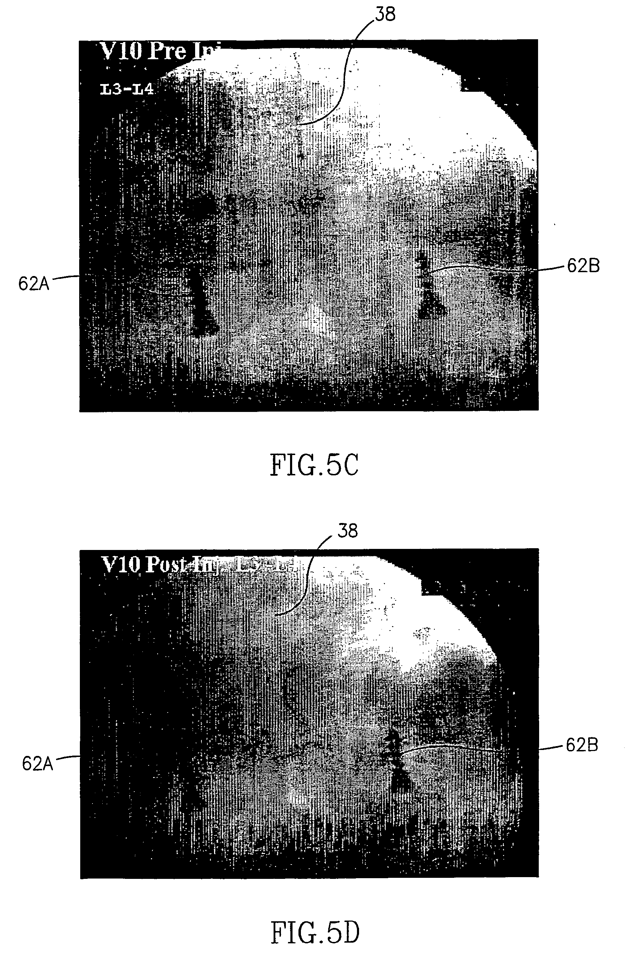

Figs. 5C and 5D represent the X-ray images in the region of Vertebrae L3 and L4 prior to and immediately after the injection of ribose cross-linked collagen into the intra-discal space, respectively. In Fig. 5C which was taken after the insertion of the injecting needle into the intervertebral disc space of the disc between vertebrae L3 and L4 but before the injection of the cross-linked collagen, part of the injecting needle 38 may be seen entering the intra-discal space from above. The screws 62A and 62B are the cancellous screws inserted into the L3 and L4 vertebrae, respectively, as disclosed in detail hereinabove.

The X-ray photograph illustrated in Fig 5D was taken immediately after the injection of approximately 1.5 milliliters of ribose cross-linked collagen having a concentration of 35 milliliters of porcine ribose cross-linked collagen into the L3-L4 intra-discal space, without moving the pig or the imaging machine during the entire injection and imaging procedure. The screws 62A and 62B are seen in Fig. 5D.

To obtain an indication proportional to the intervertebral separation (intervertebral spacing) of the L2 and L3 vertebrae and of the L3 and L4 vertebrae the distance between the opposing vertebrae was measured directly on the X-ray film and the % difference was calculated from the measured values. The results of the X-ray film measurements for pig V10 (in millimeters) are given in TABLE 4 below.

**TABLE 4**

| Spinal Level | Intervertebral separation as measured on X-ray film (millimeters) | | % change |
|---|---|---|---|
| | Before Injection | After Injection. | |
| L2 - L3 | 0.4 | 0.7 | +75 |
| L3 - L4 | 0.55 | 0.85 | +55 |

It is noted that the values in TABLE 4 above do not represent the actual values of the intervertebral separations within the pig but are proportional to these values.

The results illustrated in Figs. 5A-5D and in TABLE 4 above clearly show that intervertebral spaces in both L2-L3 and L3-L4 levels were substantially increased after ribose cross-linked collagen injection.

Four days after ribose cross-linked collagen administration, pig V10 was sacrificed. The four degenerated discs and their facet joints were processed for histological examination as disclosed hereinabove.

Pigs V11-V20 were grouped into two subgroups. Pigs V11-V14 were destined for histopathological evaluation, while pigs V15-V20 were destined for biomechanical evaluation. The degenerative model was created in three discs in the V11-V14 pig subgroup, and in two discs the V15-V20 pig subgroup.

The histopathological and biomechanical evaluations will be completed by Q1 2003.

In addition to the 6 pigs (V15-V20) in subgroups B, segments of the lumbar vertebral columns of two additional pigs having approximately the same weight and age, which were not operated, were examined for biomechanics, and used as a second control group (non-operated, non-injected) for the degenerative disc model.

Five pigs, (pigs R10-R11 and U3-U5 of TABLE 3 above) are used for safety evaluation. Except for pig R10, which was injected in the intra dural (spinal) space, all the other pigs had an epidural injection. All pigs participating in EXPERIMENT 3 were generally in a very good condition, and displayed a perfect neurological performance. Neither limping nor urination problems were observed in any of the animals before sacrificing.

### Exemplary results from Experiment 3

The mechanical tests were performed on intact L1-L5 vertebral column segments harvested ten (10) months after creating the model of disc degeneration in pig V19 and pig V16 of TABLE 3. In pig V16, disc L2-L3 and disc L3-L4 were injected with ribose cross-linked porcine collagen at the fourth month after the induction of the model DDD. The same mechanical tests were performed on a non-operated virgin vertebral column of a pig designated **control 1** (a non-operated pig having the same age and weight as the tested pigs). The three columns were tested using the INSTRON machine as disclosed in detail hereinabove. Five cycles of loading and unloading were performed for each of the three harvested columns at the physiological ranges of 40 Newtons X meter in flexion and extension, and torsion.

The results of the mechanical measurements are summarized in Figs. 6A-6F and in TABLE 6 - TABLE 8 below.

Reference is now made to Figs. 6A-6F which are exemplary schematic graphs illustrating curves representing examples of results of flexion experiments performed on dissected pig lumbar spine column segments from control (Control) and experimentally treated pigs (pigs V16 and V19), pig V16 having intervertebral discs injected with ribose cross-linked collagen, in accordance with an embodiment of the methods of the present invention.

The graphs illustrated in Figs. 6A-6C are force versus displacement graphs. In the graphs of Figs. 6A-6C, the vertical axes represent the force acting on the arm 53 (in Newtons) and the horizontal axes represent the displacement of the arms of the extensiometer 62.

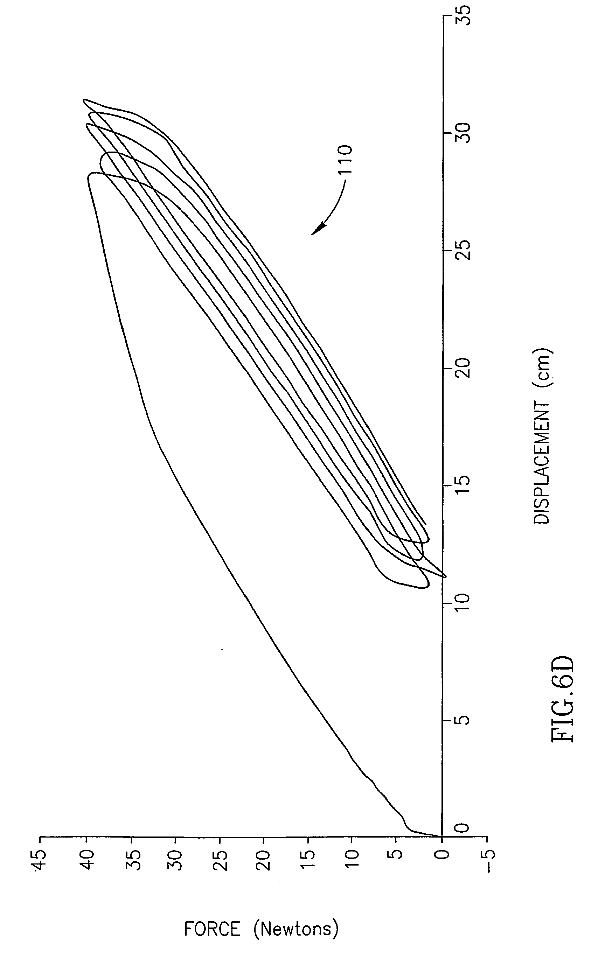

The graphs illustrated in Figs. 6D-6F are also force versus displacement graphs. In the graphs of Figs. 6D-6F, the vertical axes represent the force (in Newtons) acting on the arm 65 due to the movement of the bridge 60, and the horizontal axes represent the displacement of the bridge 60 (wherein zero displacement arbitrarily indicates the position of the bridge 60 at the beginning of the test cycles.

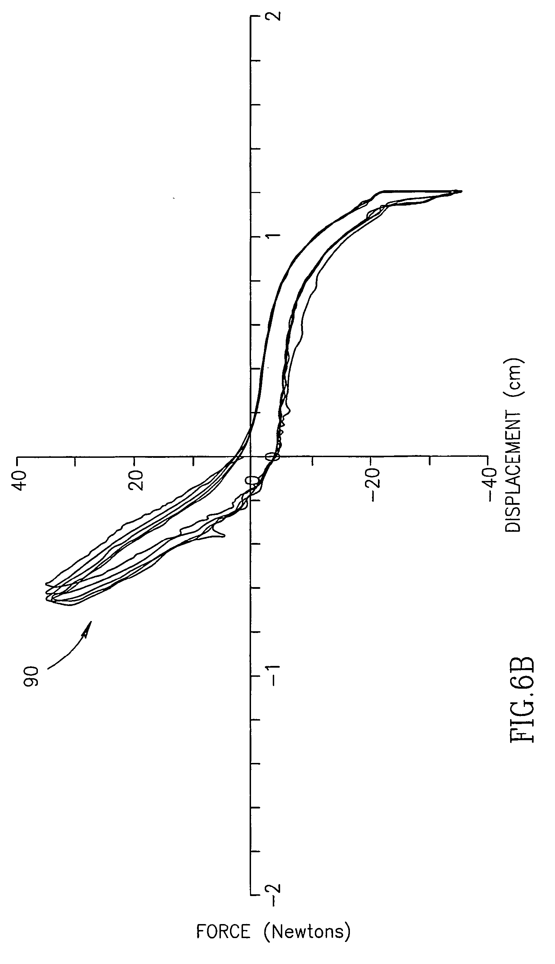

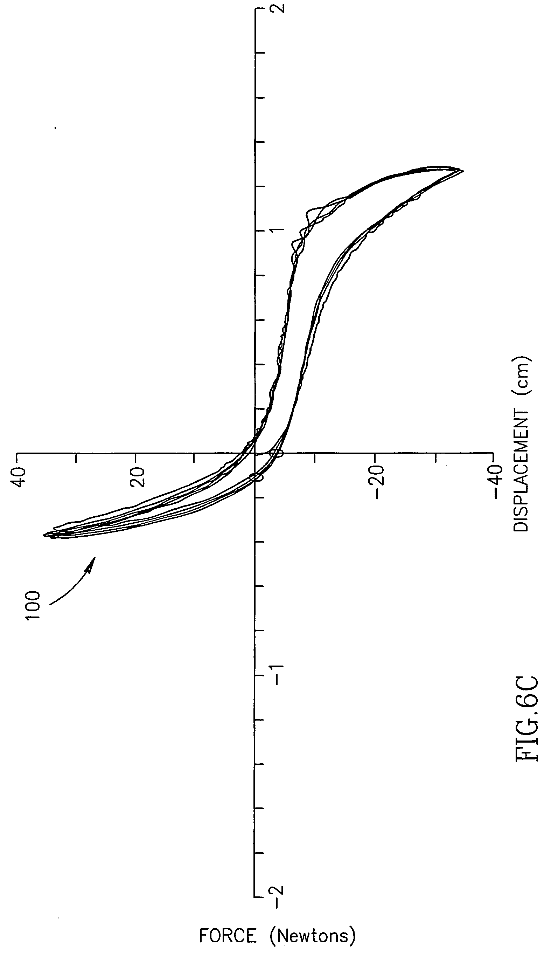

In Fig. 6A, the curve 80 represent the Force versus Displacement data for the five cycles of flexion measurements performed in the spinal column from the control1 pig. In Fig. 6B, the curve 90 represent the Force versus Displacement data for the five cycles of flexion measurements performed in the spinal column from the pig V16. In Fig. 6C, the curve 100 represent the Force versus Displacement data for the five cycles of flexion measurements performed in the spinal column from the pig V19. The displacement values are given in centimeters.

Turning to Fig 6A, the curve 80 has three parts. The middle part 80A of the curve 80 is typically nearly horizontal or has a relatively shallow slope, which represents the region of bending (flexion or extension) in which the application of a relatively small force results in a relatively large vertebral displacement, representing a relatively large flexion or extension of the spinal column segment being tested. The side parts 80B and 80C of the curve 80 represent movement regions having a relatively large slope, in which regions the application of larger forces are needed to induce a displacement than in the middle region 80A of the curve 80.

Typically, in degenerative inter-vertebral discs, the range of displacement spanned by the (linear or nearly linear) middle part of the force versus displacement curve increases as compared to a comparable non-degenerative disc.

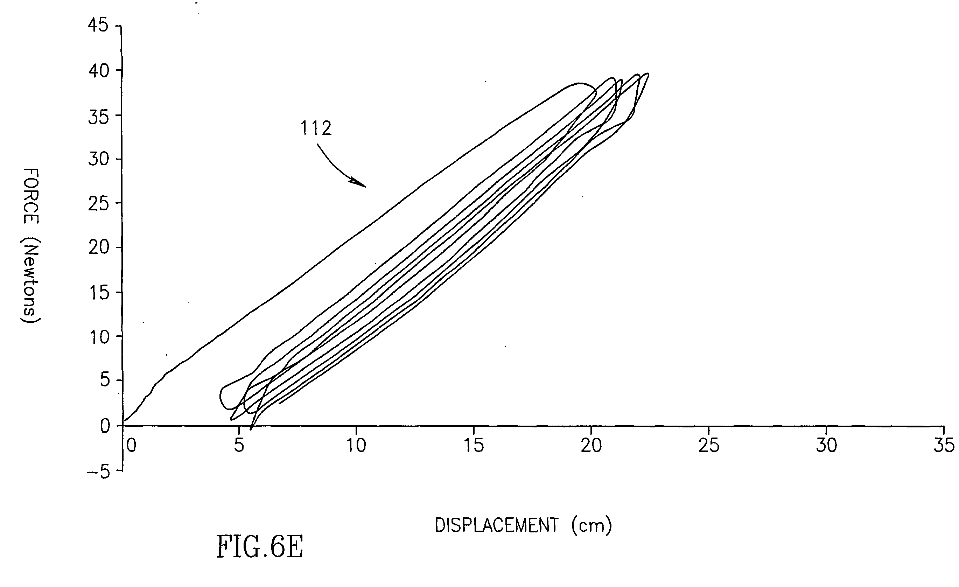

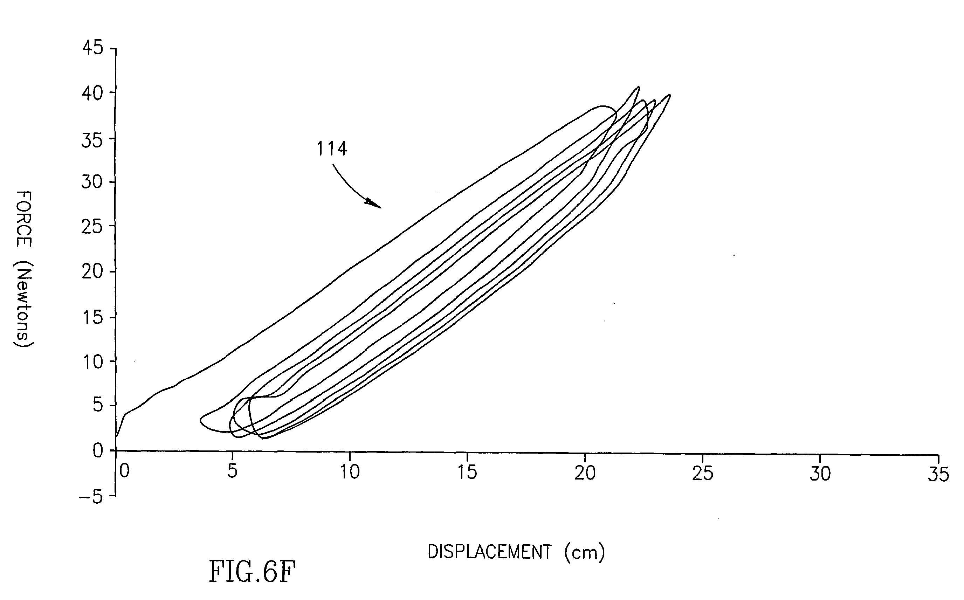

In Fig. 6D, the curve 110 represent the Force versus Displacement data for the five cycles of torsion measurements performed in the spinal column from the control1 pig. In Fig. 6E, the curve 112 represent the Force versus Displacement data for the five cycles of torsion measurements performed in the spinal column from the pig V16. In Fig. 6F, the curve 114 represent the Force versus Displacement data for the five cycles of torsion measurements performed in the spinal column from the pig V19. The displacement values represent the displacement in centimeters of the bridge 60 from an arbitrary zero point representing the position of the bridge 60 at the beginning of the test, as disclosed in detail hereinabove.

Turning to Fig. 6D, the curve 110 represents the displacement of the bridge 60 versus the force loading the arm 65 (to induce torsion in the lumbar spinal column segment being tested, as disclosed in detail hereinabove (in Fig. 6D the spinal column segment is from the control1 pig).

**TABLE 6: Measurements of Torsion (range of displacement)**

| Pig designation | Bridge Displacement range (cm) |
|---|---|
| Control1 | 18.8 |
| V16 | 18.4 |
| V19 | 19.4 |

The measurement of the results of the torsion tests shown in TABLE 6 were performed on a parts the curves 110, 112 and 114 illustrated in Figs. 6D, 6E and 6F, respectively.

For each of the curves 110, 112 and 114, two points on the curve representing the reversal of bridge movement at the beginning and the end of the third torsion cycle were identified and marked on the curve. The values of displacement of the bridge 60 for these two points (the points are not shown on the curves for the sake of clarity of illustration) were determined on the displacement axis and the bridge displacement range was determined by subtracting the displacement value at the point corresponding to the end of the third torsion cycle from the value of the displacement at the point corresponding to the beginning of the third torsion cycle.

**TABLE 7: Measurements of flexion and extension (total range of displacement).**

| Pig designation | Total Extensiometer Displacement Range (cm) |
|---|---|
| Control1 | 2.0 |
| V16 | 1.8 |
| V19 | 1.66 |

The measurement of the results of the flexion and extension tests shown in TABLE 7 were performed on the curves 80, 90 and 100 illustrated in Figs. 6A, 6B and 6C, respectively.

For each of the curves 80, 90 and 100, The following procedure was used to determine the total extensiometer displacement range. The procedure is explained for the particular example of curve 80 of Fig. 6A. Turning to Fig. 6A, the point 85 represents the average position of the five points representing the reversal of the direction of movement of the arms of the extensiometer 62 for each cycle of the five cycles in the upper left quadrant of the graph illustrated in Fig. 6A. Similarly, the point 87 represents the average position of the five points representing the reversal of the direction of movement of the arms of the extensiometer 62 for each cycle of the five cycles in the lower right quadrant of the graph illustrated in Fig. 6A. The position of the points 85 and 87 was visually estimated. The displacement values corresponding to the horizontal axis coordinate of each of the points 85 and 87 were determined, and the value of the displacement for point 85 was subtracted from the displacement value for point 87 to give_the value of the total extensiometer displacement range for the curve 80 of Fig. 6A. This value represents the total (non-linear) range of displacement under the experimental force used.

For the curves 90 and 100 of Figs. 6B and 6C, respectively the same measurement procedure as disclosed in detail for the curve 80 of Fig. 6A was used, and the values of the total extensiometer displacement range are given in TABLE 7.

In TABLE 8 below, the results of measurements of the middle nearly linear part of the uppermost and lowermost flexion curves for the pigs control1, V16 and V19, respectively, are shown. The range of displacement is given in centimeters.

**TABLE 8:**

| Pig designation | Extensiometer Displacement range for Upper cycle portions (cm) | Extensiometer Displacement range for Lower cycle portions (cm) |
|---|---|---|
| Control1 | 0.56 | 0.54 |
| V16 | 0.6 | 0.58 |
| V19 | 0.66 | 0.62 |

The measurement of the results of the flexion and extension tests shown in TABLE 8 were performed on the curves 80, 90 and 100 illustrated in Figs. 6A, 6B and 6C, respectively.

For each of the curves 80, 90 and 100, the following procedure was used to determine the extensiometer displacement range for the upper and lower middle portions of the nearly linear parts of the experimental curves 80, 90 and 100. The procedure is explained for the particular example of curve 80 of Fig. 6A. Turning to Fig. 6A, the displacement range over which the force/displacement curve was nearly linear was visually estimated from all five curve portions 80AU in the upper part of the curve 80. Similarly, the displacement range over which the force/displacement curve was nearly linear was visually estimated from the four curve portions 80AL in the lower_part of the curve 80. The first cycle portion 87 was ignored since it represents part of the first cycle of the curve 80 at which the hysteresis behavior of the curve 80 has not yet stabilized.

The displacement values corresponding to the horizontal axis coordinates corresponding to the average end points (nor shown) of the group of the upper nearly linear portion group **80AU** were visually determined, and the range of the nearly linear extensiometer displacement over the upper cycle portions was determined by subtracting the value of one end point displacement value from the value of the remaining end point. Similarly, the displacement values corresponding to the horizontal axis coordinates corresponding to the average end points (nor shown) of the group of the lower nearly linear portion group **80AL** were determined, and the range of the nearly linear extensiometer displacement over the upper cycle portions was determined by subtracting the end point displacement values as disclosed hereinabove.

The values thus obtained, represent the range of displacement for the nearly linear middle region of spinal column movement under the experimental force used.

For the curves 90 and 100 of Figs. 6B and 6C, respectively, the same measurement procedure as disclosed in detail for the curve 80 of Fig. 6A was used, and the values of the range of displacement for the nearly linear middle region of extensiometer displacement range were determined for the upper and lower groups of curve portions as disclosed for curve 80 hereinabove and the values obtained are given in TABLE 8 above.

It can be seen that there is no significant difference between the torsion results of pigs V16 and V19. The range of motion of the vertebral columns of pigs V16 and V19 is less than that for the control pig (pig control1).

For the flexion and extension study, the range of displacement for the column of pig V16 is slightly larger than that of the control pig (pig control1) and the displacement range for the column segment of pig V19 is larger than the displacement ranges of the pig control1 and pig V16. The range of instability as is measured by the length of the linear middle part of the curves is somewhat larger in pig V16 than that in the control1 pig, and is even larger in pig V19 than in pig V16.

From the clinical point of view these results show preservation of flexional and torsional mechanical properties of the column of the pigs which received the therapeutic injection of cross-linked collagen into the intra-discal space of model degenerate discs.

The flexion and torsion results shown above and illustrated in Figs. 6A-6F indicate that in accordance with an embodiment of the present invention, it may be possible to therapeutically preserve at least some of the mechanical properties of degenerated discs in a mammal by intra-discal injection of an injectable preparation of ribose cross-linked collagen.

### EXPERIMENT 4

This experiment was performed to determine the relation between the pressure at the outlet of the syringe 33 (Fig. 3) and the pressure within the inner disc space of the disc 30 (Fig. 3) at the end of the injection of cross linked collagen.

Three virgin pigs (numbered as Pig 1, Pig 2 and Pig 3) having approximately the same weight as Pig V10 of EXPERIMENT 3 were sacrificed. The lumbar vertebral columns of all three were harvested as disclosed hereinabove and the discs at the L2-L3 and at the L3-L4 of each lumbar vertebral column segment were injected with 1.5 milliliters of porcine ribose cross-linked collagen injectable preparation having a concentration of 35 milligrams/milliliter of PBS. The system 20 (illustrated in Fig. 3) was used for performing the injection and pressure measurements as disclosed in detail hereinabove.

The pressure at the outlet of the syringe 33 was measured as disclosed hereinabove by the pressure sensing unit 26 (Fig. 3) at the end of the injection. The pressure in the inner disc space was measured as disclosed hereinabove by the pressure sensing unit 28 (Fig. 3) at the end of the injection.

The results of the pressure measurements are summarized in TABLE 9 below.

**TABLE 9**

| Spinal Level Of injected disc | Pressure at outlet of syringe at the end of the injection (Atmospheres) | | | Intra-discal Pressure at the end of the injection (Atmospheres) | | |
|---|---|---|---|---|---|---|
| | Pig 1 | Pig 2 | Pig 3 | Pig 1 | Pig 2 | Pig 3 |
| L2 - L3 | 12 | 10 | 9 | 7.5 | 7 | 6.5 |
| L3 - L4 | 10 | 9 | 11 | 6 | 6 | 5.5 |

The results in TABLE 9 above indicate that outlet pressures in the range of 9-12 atmospheres may be used in the injection system 20 of the present invention without causing disc rupture. The application of such pressures at the outlet of the syringe 33 resulted in the development of intra-discal pressure levels within the range of 5.5-7.5 atmospheres.

It is noted that in the injections performed in EXPERIMENT 3 and EXPERIMENT 4, when a 22G needle was used for injecting the intervertebral discs, the force applied to the rod 35 of the syringe 33 did not exceed 450 Newtons. In all these experiments, there was no observable leakage of the injected cross-linked collagen material from the point of entry of the 22G needle into the annulus fibrosus of the injected disc. Furthermore, after the injection needle was withdrawn from the disc (at the end of the pressure injection of the collagen preparation), no leakage of the collagen preparation was observed from the punctured region of the annulus fibrosus, indicating a good seal of the puncture made by the injection needle which prevented leakage of intra-discally injected material.

The results of the experiments disclosed hereinabove and illustrated in the drawings indicate that the intra-discal injection of cross-linked collagen preparations, and preferably preparations containing injectable reconstituted atelopeptide fibrillar type-I collagen cross-linked by D(-)ribose (or by other suitable reducing sugars) into one or more discs of the spinal column, may be used in human patients as a therapeutic procedure for treating human patients presenting degenerative disc disease (DDD) symptoms, or as a preventive treatment in patients which may be expected to be susceptible to DDD. The injection method may be used, inter alia, to improve or restore one or more mechanical properties of the spinal column including, but not limited to, the distance or spacing between vertebrae flanking the injected disc(s), the flexional and/or torsional mechanical properties of the spinal column, or other mechanical properties of the injected disc(s) or of the spinal column of the patient. Such therapy as disclosed herein may be used ,inter alia, to increase the height of the patient by increasing the distance between the vertebrae flanking the injected disc(s).

An advantage of the present invention is that due to the volumes injected into the disc, the specific pressure range used for injection, and to the rheological properties of the injected material, the increase in intervertebral spacing and the improvement or restoration of spinal biomechanical properties is effected immediately after injecting the disc(s) and does not depend (at least for the short term effect of the injection) on the in-vivo induction or buildup of proteoglycans, or on the growth or restoration of nucleus pulposus tissue. It is, however noted that long term effects of the treatment method disclosed herein may also include the *in situ* deposition or production of proteoglycans within the intra-discal space or within the injected cross-linked collagen material injected into the treated disc, and may also include contributions due to longer term growth or restoration of nucleus pulposus tissue within the intra-discal space.

In applying the injectable fluid medicament in human patients, the volume and concentrations of the injected cross-linked collagen preparations may be similar to the experimental values used in pigs. The intra-discal pressure level within the injected human disc may preferably be in the range of 0.5-12 atmospheres, and more preferably in the range of the injected volume used may be in the range of 0.5-2.5 milliliters, and the collagen concentration is comprised between 35 and 55 milligrams of cross-linked collagen per milliliter of injectable preparation.

It is, however, noted that the above preferred values may be modified or adapted, depending, inter alia, on the patients age, the size and type of the disc(s) to be injected, and the species of the mammal which is being treated. For Example, if the method is used to treat canine DDD, some or all of the treatment parameters, such as ,inter alia, the volume of collagen injected, the injection pressure needed to inject the desired volume of cross-linked collagen within a reasonably practical time, the gauge of the needle, may need to be suitably adapted.

### Preparation of injectable ribose cross linked collagen

The injectable cross-linked collagen used in EXPERIMENT 1 was an injectable sterile ribose cross-linked porcine collagen preparation comprising 35 milligram reconstituted atelopeptide porcine collagen cross linked by ribose per milliliter of injectable preparation. The cross-linked collagen was suspended in PBS. Methods of preparation of various forms of ribose cross linked collagen are also disclosed in detail in International Patent Application, PCT/IL01/00351, published as international publication number WO 01/79342 A2.

Briefly, a solution of molecular purified pepsinized atelopeptide porcine Type I collagen (1-10 milligram/milliliter) was prepared from porcine (pig) tendons commercially available from Pel-Freeze®, Arkanssas LLC, AR, U.S.A, by pepsinization in the presence of acetic acid and purification by repeated salt precipitation, as is known in the art. (see also U.S. Patent 5,955,438 to Pitaru et al. and U.S. Patent 4,971,954 to Brodsky et al. The resulting molecular purified pepsinized atelopeptide porcine Type I collagen was dissolved in 0.01M HCI and maintained at 4°C. The cold acidic pepsinized collagen solution (at pH 3 and a collagen concentration of 3 mg/ml) is neutralized to pH 7.2-7.4 with an alkali phosphate buffer (comprising 1.6 grams sodium hydroxide and 35.6 grams of disodium hydrogen phosphate dihydrate per 1liter of buffer and having a pH of approximately 11.2), warmed to 37°C and vigorously stirred and shaken on a shaker for 18-24 hours.

The continuous stirring results in the formation of small particles in the range of approximately 30-300 micron. The fibrillar collagen particulate matrix obtained after 24 hours of incubation at 37°C is centrifuged to precipitate the collagen particles (typically, this incubation may be performed at a temperature in the range of approximately 4°C-37°C) . The supernatant is removed and the particulate collagen pellet washed several times in phosphate buffered saline (PBS) by repeated centrifugation and re-suspension.

The pellet spun down in the last washing may be resuspended in a 0.5-80% (w/v) D(-)Ribose solution in PBS. The volume of ribose solution used is approximately two times the volume of the spun down particulate collagen pellet. The resulting suspension is injected forcefully under pressure through an injecting device (such as a hollow stainless steel tube or needle) into 100% ethanol.

The stainless steel tube may be in the range of 14 gauge to 34 gauge, but other suitable tube sizes may also be used.

Preferably, the volume of the ethanol into which the above described collagen/ribose/PBS mixture is injected, and the exact concentration of the D(-)Ribose in PBS are selected such that after the injection is completed the final concentration of the D(-) ribose in the final mixture is approximately 1% (w/v), and the concentration of ethanol in the final mixture is 70% (v/v). It is noted that these concentrations are given by way of example only and other concentrations of D(-) ribose and/or ethanol may also be used, as disclosed in detail in international publication number WO 01/79342 A2. Furthermore, it may also be possible to use other sugar types for cross-linking the collagen as disclosed in detail in international publication number WO 01/79342 A2.

The injecting under pressure is performed in order to achieve proper dispersion of the small reconstituted fibrillar collagen particles in the cross-linking solution and to prevent aggregation of collagen particles due to the dehydrative action of the ethanol. The collagen suspension is then mixed and/or shaken for approximately 3 -14 days at a temperature in the range of 4°C-45°C for allowing the cross-linking of collagen to occur (other temperatures may also be used).

The resulting cross-linked collagen suspension is washed several times in phosphate buffered saline (PBS) by repeated centrifugation and re-suspension to remove the unreacted ribose and the ethanol. Then, the pellet is resuspended in PBS and allowed to equilibrate overnight for rehydration at 37°C (this equilibration step may be typically performed at temperatures in the range of approximately 4°C-37°C, but other temperatures may also be used). The collagen is then washed two more times in PBS as disclosed hereinabove and resuspended in PBS to a final concentration in the range of 15-60 milligrams cross-linked collagen per milliliter of suspension. This porcine preparation was used for injecting into the porcine intervertebral discs as disclosed hereinabove in EXPERIMENT 1, EXPERIMENT 3 and EXPERIMENT 4, and for injecting into the rabbit ears as disclosed hereinabove in EXPERIMENT 2. Typically, a concentration of approximately 25-35 milligrams cross-linked collagen per milliliter of suspension was used for rabbit ear injections and a range of 35-55 milligrams per milliliter cross-linked collagen was used for intra-discal injection experiments.

Additionally, it is noted that the liquid in which the collagen preparation is suspended is not limited to PBS but may be any suitable type of biologically tolerable and pharmaceutically acceptable buffer or injectable fluid known in the art.

An injectable sterile ribose cross-linked bovine collagen preparation was prepared as disclosed hereinabove for the porcine preparation, except that instead of porcine tendons, bovine tendons were used. The bovine tendons are commercially available from Pel-Freez®, Arkanssas LLC, Rogers, AR, U.S.A. The concentration of the cross-linked bovine collagen used was 25-35 milligrams cross-linked bovine collagen per milliliter of suspension.

It is noted that the method and preparations of the present invention may also be used to deliver into the treated intervertebral disc specific substances (such as but not limited to local growth factors) or living cells (such as but not limited to chondrocytes) which may have a therapeutic effect such as, but not limited to inducing bone formation, fibrosis or cartilage formation or the formation of other tissue types.

In accordance with other embodiments of the present invention, the injectable cross-linked collagen preparations used for treating intervertebral discs may be modified. For example, the injectable cross-linked collagen preparations may include additional substances, living cells, drugs, therapeutic materials or compounds or agents, and genetic material for gene therapy.

The living cells which may be added to the injectable cross-linked collagen preparation of the invention may include, inter alia, vertebrate chondrocytes, vertebrate stem cells, vertebrate progenitor cells, vertebrate fibroblasts, genetically engineered cells that are engineered to secrete matrix proteins, glycosaminoglicans, proteoglycans, morphogenic proteins, growth factors, transcription factors, anti-inflammatory agents such as proteins, hormones or peptides, or cells that have been engineered to express receptors to molecules such as proteins, glycosaminoglicans, proteoglycans, morphogenic proteins, growth factors, transcription factors, anti-inflammatory agents such as proteins, hormones, peptides, various different transcription factors, or cells that express any combination of the above described secreted substances and/or the above described receptors.

Other substances or compounds which may be included in the injectable cross-linked collagen preparation of the invention may include, inter alia, anaesthetic compounds or agents, anti-inflammatory compounds, agents or drugs, vertebrate growth factors proteins, glycosaminoglicans, proteoglycans, morphogenic proteins, anti-inflammatory agents such as proteins, hormones, peptides, and various transcription factors.

Other substances or compounds which may be included in the injectable cross-linked collagen preparation of the invention may include, inter alia, a nucleic acid, an oligonucleotide, ribonucleic acid, deoxyribonucleic acid, a chimeric DNA/RNA construct, DNA or RNA probes, anti-sense DNA, anti-sense RNA, a gene, a part of a gene, a composition including naturally or artificially produced oligonucleotides, a plasmid DNA, a cosmid DNA, or any other substance or compound containing nucleic acids or chemically modified nucleic acids, or various combination or mixtures of the above disclosed substances, compounds and genetic constructs, and may also include the vectors required for promoting cellular uptake and transcription, such as but not limited to various viral or non-viral vectors known in the art.

Additionally, other substances or compounds which may be included in the injectable cross-linked collagen preparation of the invention may include, inter alia, various proteins, glycoproteins, mucoproteins, mucopolysaccharides, glycosaminoglycans such as but not limited to chondroitin 4-sulfate, chondroitin 6-sulfate, keratan sulfate, dermatan sulfate, heparin, heparan sulfate, hyaluronan, proteoglycans such as the lecitin rich interstitial proteoglycans decorin, biglycan, fibromodulin, lumican, aggrecan, syndecans, beta-glycan, versican, centroglycan and serglycin, fibronectins, fibroglycan, chondroadherins, fibulins, and thrombospondin-5.

Additional substances and compounds which may be also included in the collagen preparations of the present invention may be, inter alia, various different enzymes, various different enzyme inhibitors, and antibodies.

The adding of the various different substances, therapeutic agents, compounds or cells to the cross-linked collagen preparation may be achieved using different methods. In accordance with one embodiment of the present invention, one or more of the substances or compounds disclosed hereinabove may be added to the collagen suspension or solution prior to the injection of the collagen and D(-) Ribose mixture into the ethanol. In this embodiment the cross-linking is performed in the presence of the added substance, compound or therapeutic agent, which may become trapped or sequestered within the resulting cross-linked collagen based matrix.

In accordance with another embodiment of the invention, the substances, therapeutic agents, compounds or cells may be added to or mixed with a suspension of the cross-linked collagen matrix by physical mixing.

Preferably, when live cells are added to the injectable cross-linked collagen preparation, they are added by suitable gentle mixing of the washed ribose cross-linked collagen injectable preparation (prepared with or without additional substances as disclosed hereinabove) in order to avoid contact of live cells with concentrated ethanol.

The advantages of using injectable cross-linked collagen preparations are several. The resistance of ribose cross-linked collagen to in-vivo biodegradation by naturally occurring collagenolytic enzymes, is much higher than in native collagen. It is therefore expected that the discs injected with the cross-linked collagen preparations may be less affected by such biodegradation while still being highly biocompatible.

Another advantage is that the collagen (cross-linked or non-cross-linked) may serve as a matrix for inducing and/or promoting the formation of cartilage or fibrocartilage in the disc. This is supported by the results of EXPERIMENT 2.

It is noted that while the ribose cross linked collagen used for intervertebral disc repair was derived from purified bovine and porcine atelopeptide collagen, many other types of collagen may be used in the disc repair and stabilization methods of the present invention, including but not limited to cross-linked and non-cross-linked collagen preparations obtained from other vertebrate species, including human collagen, cross-linked and non-cross-linked collagen preparations derived from recombinant collagen, or any other suitable types of genetically engineered or modified collagen.

It is further noted that although the ribose cross-linked collagen preparation was selected for use in disc repair due to its superior resistance to biological degradation in situ, it may also be possible to use non cross-linked collagen preparations in the same manner disclosed, though they may be degraded faster.

Additionally, it may be possible to use various mixtures of cross-linked and non-cross-linked collagen in the methods of intervertebral disc repair of the present invention, which may enable better control of the degradation resistance properties and other physical or chemical properties of the mixture. This may be particularly useful when live cells such as chondrocytes or other cells are added to the collagen preparation to promote tissue formation in situ (such as for example the formation of cartilage or fibrocartilage tissue). The mixing of different collagen types may enable better control of the cell proliferation and tissue formation by suitable modifications of the collagen mixture composition.

Additionally, it may be possible to use mixtures of various different collagen types, such as but not limited to, collagen types I, II and IX, or any other suitable mixture of any other types of collagen known in the art.

Furthermore, the methods and preparations of the present invention may make use of or may include, respectively, artificially produced collagen types which are manufactured by genetically modified eukaryotic or prokaryotic cells or by genetically modified organisms, as is known in the art.

Moreover, while in the experiments disclosed in the present invention the preferred material used for intra-discal injection is injectable suspensions of reconstituted fibrillar atelopeptide porcine collagen cross-linked with ribose as disclosed in detail hereinabove, the method of injecting of non-herniated mammalian discs of the present invention may also be performed by using other different injectable preparations. For example, suitable injectable preparations may include but are not limited to, injectable preparations comprising a suspension of particles of cross-linked hyaluronic acid or hyaluronic acid derivatives or hyaluronic acid salts or gels derived therefrom or cross-linked forms thereof, or hyaluronan and derivatives thereof, as is known in the art. Such materials and similar materials may have water retaining properties, as well as other biocompatibility an non-alergenic properties, and rheological properties which are desirable in the present methods for treating intervertebral discs. Such materials and similar materials may therefore be advantageously used for the intervertebral disc injection methods of the present invention.

For example, Injectable hyaluronic acid gel may be used for soft tissue augmentation. ( see the article entitled "SAFETY DATA OF INJECTABLE NONANIMAL STABILIZED HYALURONIC ACID GEL FOR SOFT TISSUE AUGMENTATION. "by Friedman PM, Mafong EA, Kauvar AN, Geronemus RG., Dermatol. Surg. 2002 Jun;28(6):491-4.

### Cross-linked collagen implants for damaged or herniated intervertebral discs

It is noted that the advantages of the biocompatible collagen material may also be utilized in damaged, herniated or fissured intervertebral discs. In accordance with another preferred embodiment of the present invention, a collagen based implant is surgically introduced into the intervertebral disc after surgical removal of part of the material of the nucleus pulposus or of the entire nucleus pulposus. The removal of the nucleus pulposus may be performed using any surgical method known in the art for removal of part of or the entire nucleus pulposus.

The implant may comprises one or more pieces comprising dry collagenous material. Preferably, the collagenous implant or implants may comprise ribose cross-linked collagen due to its superior biodegradation resistance in vivo, but other types of collagenous materials may also be used. The collagenous implant(s) may act as space maintainers for stabilization of the damaged intervertebral disc and may also promote the formation of fibrocartilage tissue in situ which may further contribute to the long term biomechanical stabilization of the treated disc.

After the introduction of the dry collagen based implant or implants into the chamber previously occupied by the nucleus pulposus of the treated intervertebral disc, the dry implant(s) expand by rehydration. The size and shape of the implants may be adapted such that after dehydration and swelling they fill most of the space inside the treated disc after the surgical removal of the nucleus pulposus to provide mechanical support and stabilization of the intervertebral disc shape contributing to vertebral column stabilization and preventing further vertebral damage.

Due to the relatively small size of the dry swellable collagen based implants prior to swelling it may be possible to introduce the implants into the treated intervertebral disc through a small opening surgically made in the annulus fibrosus. Such methods for accessing the interior of the intervertebral disc for removal and/or introduction of materials are known in the art. For example, U.S. Patent 6,099,514 to Sharkey discloses method and apparatus for delivering or removing material from the interior of an intervertebral disc. U.S. Patent 6,264,695 to Stoy, discloses a spinal nucleus implant made from a biomimetic xerogel plastic and methods for its introduction into an intervertebral disc.

The swellable cross-linked collagen implants of the present invention, may be introduced into the intervertebral disc in a dry or partially hydrated form by suitably modifying the methods disclosed by Sharkey or Stoy or other methods or devices known in the art for the introduction of intervertebral disc implants.

The cross-linked collagen based implants may be adapted to have various shapes after the rehydration induced in-situ swelling. For example the implant(s) may have a disc like shape or any other shape suitable for a disc implant which is known in the art, such as but not limited to, the implant shapes disclosed by Stoy (U.S. patent 6,264,695).

The cross-linked collagen material included in the swellable disc implants may be prepared as disclosed in U.S. Patent 5,955,438 to Pitaru et al., U.S. Patent 4,971,954 to Brodsky et al, and in international publication number WO 01/79342 A2.

The implants of the present invention may include various different compounds and/or substances and/or additives and/or pharmaceutical compositions or therapeutic agents, and/or drugs, as disclosed in detail hereinabove for the injectable cross-linked collagen based preparations.

It is noted that while live cells are typically not included in dry swellable implants, they may be included in semi-dry or partially hydrated implants. Nevertheless, such living cells may be introduced into the implant after partial or complete hydration by irrigating the implant with a suitable physiological solution or a liquid medium containing living cells of the types disclosed hereinabove in connection with the injectable collagen preparations.

The advantages of the swellable collagen implants are that they are biocompatible, they may be inserted into the disc through a relatively small opening due to their small volume in the dry or in the partially hydrated state. Additional, in the case of implants including ribose cross-linked collagen an additional advantage is the high resistance to biodegradation in vivo.

A further advantage of the collagen based implants of the present invention is that even without the addition of isolated living chondrocytes, they may induce migration of the chondrocytes locally present in the disc and may encourage and induce fibrocartilage formation inside the disc as disclosed hereinabove and as demonstrated by the results of EXPERIMENT 2 above.

It is noted that while the method and preparations for treating intervertebral discs may be adapted to treat humans, the method and compositions of preparations may be applied similarly or with suitable modifications to treat intervertebral disc disease in other mammals or even in other vertebrates. For example, canine IVD is a known problem in certain dog breeds. The methods and injectable preparations disclosed may thus be applied to dogs or other pets or domestic or domesticated animals, as disclosed hereinabove.

## Claims

1. Use of collagen cross-linked with a reducing sugar for the manufacture of an injectable fluid medicament for treatment of degenerate disc disease of a vertebral column, wherein the concentration of said collagen in said medicament is comprised between 35 and 55mg per milliliter of medicament.

2. The use of collagen cross-linked with a reducing sugar according to any of the preceding claims, wherein said sugar is D(-) ribose.

3. The use of collagen cross-linked with a reducing sugar according to any of the preceding claims wherein said medicament further comprises a pharmaceutically acceptable buffer or a pharmaceutically acceptable injectable fluid.

4. The use of collagen cross-linked with a reducing sugar according to any of the preceding claims, wherein said collagen is selected from the group consisting of bovine atelopeptide collagen, porcine atelopeptide collagen, collagen obtained from a vertebrate species, human collagen, recombinant collagen, genetically engineered or modified collagen, types I collagen, type II collagen, type IX collagen, artificially produced collagen manufactured by genetically modified eukaryotic or prokaryotic cells or by genetically modified organisms, purified collagen and purified pepsinized atelopeptide porcine Type I collagen, particles of fibrillar collagen, fibrillar reconstituted atelopeptide collagen, and combinations thereof.

5. The use of collagen cross-linked with a reducing sugar according to any of the preceding claims, wherein said vertebral column is selected from a mammalian vertebral column, a human vertebral column and a canine vertebral column.

6. The use of collagen cross-linked with a reducing sugar according to any of the preceding claims, wherein said medicament is effective in restoring and/or preserving at least one mechanical property of the vertebral column or a part thereof, said restoring and/or preserving comprises at least one of the following effects:
- increasing the spacing between the two vertebrae flanking an intervertebral disc of said vertebral column,
- at least partially restoring the flexional mechanical properties of an intervertebral disc of said vertebral column,
- at least partially restoring the torsional mechanical properties of an intervertebral disc of said vertebral column,
- at least partially maintaining the spacing between the two vertebrae flanking an intervertebral disc of said vertebral column,
- at least partially maintaining after the injection of said medicament the flexional mechanical properties of an intervertebral disc of said vertebral column,
- at least partially maintaining after the injection of said medicament the torsional mechanical properties of an intervertebral disc of said vertebral column,
- inducing in vivo formation of fibrocartilage in the internal space of said intervertebral disc or in the nucleus pulposus of an intervertebral disc of said vertebral column,
- inducing development of one or more of cartilaginous tissue, fibrocartilaginous tissue, and dense fibrous tissues in the internal space of said intervertebral disc or in the nucleus pulposus of an intervertebral disc of said vertebral column.

7. The use of collagen cross-linked with a reducing sugar according to any of the preceding claims wherein said restoring also results in at least partial relief of back pain.

8. The use of collagen cross-linked with a reducing sugar according to any of the preceding claims wherein said medicament further includes one or more ingredients selected from non-cross-linked collagen, hyaluronic acid, cross-linked hyaluronic acid, hyaluronan, a drug, a therapeutic agent, an anaesthetic agent, an anti-inflammatory agent, glycosaminoglicans, proteoglycans, morphogenic proteins glycoproteins, mucoproteins, mucopolysaccharides, growth factors, transcription factors, anti-inflammatory agents, proteins, peptides, hormones, genetic material for gene therapy, a nucleic acid, a chemically modified nucleic acid, an oligonucleotide, ribonucleic acid, deoxyribonucleic acid, a chimeric DNA/RNA construct, DNA or RNA probes, anti-sense DNA, anti-sense RNA, a gene, a part of a gene, a composition including naturally or artificially produced oligonucleotides, a plasmid DNA, a cosmid DNA, viral and non-viral vectors required for promoting cellular uptake and transcription, a glycosaminoglycan, chondroitin 4-sulfate, chondroitin 6-sulfate, keratan sulfate, dermatan sulfate, heparin, heparan sulfate, hyaluronan, a lecitin rich interstitial proteoglycan, decorin, biglycan, fibromodulin, lumican, aggrecan, syndecans, beta-glycan, versican, centroglycan, serglycin, a fibronectin, fibroglycan, chondroadherins, fibulins, thrombospondin-5, an enzyme, an enzyme inhibitor and an antibody.

9. The use of collagen cross-linked with a reducing sugar according to any of the preceding claims wherein said medicament further includes one or more types of living cells.

10. The use of collagen cross-linked with a reducing sugar according to any of the preceding claims wherein said medicament further includes one or more types of living cells and wherein said living cells are selected from vertebrate chondrocytes, vertebrate stem cells, vertebrate progenitor cells, vertebrate fibroblasts, cells genetically engineered to secrete one or more of matrix proteins, glycosaminoglicans, proteoglycans, morphogenic proteins, growth factors, transcription factors, anti-inflammatory agents, proteins, hormones, peptides, and cells that express any combinations thereof, wherein said stem cells are not human embryonic stem cells.

11. The use of collagen cross-linked with a reducing sugar according to any of the preceding claims wherein said medicament further includes one or more types of living cells engineered to express receptors to one or more molecules selected from the group consisting of proteins, peptides, hormones, glycosaminoglicans, proteoglycans, morphogenic proteins, growth factors, transcription factors, anti-inflammatory agents, and cells that express any combinations of said receptors.

12. The use of collagen cross-linked with a reducing sugar according to any of the preceding claims wherein said medicament further includes one or more types of living cells and wherein said living cells are selected from:
- cells genetically engineered to secrete one or more of substances selected from matrix proteins, glycosaminoglicans, proteoglycans, morphogenic proteins, growth factors, transcription factors, anti-inflammatory agents, proteins, hormones, and peptides,
- cells engineered to express receptors to one or more molecules selected from the group consisting of proteins, peptides, hormones, glycosaminoglicans, proteoglycans, morphogenic proteins, growth factors, transcription factors, and anti-inflammatory agents, and
- cells that express any combination of said receptors and said substances.

## Patentansprüche

1. Verwendung von Kollagen, quervernetzt mit einem reduzierenden Zucker für die Herstellung eines injizierbaren flüssigen Medikaments zur Behandlung einer degenerativen Bandscheibenerkrankung einer Wirbelsäule, wobei die Konzentration des genannten Kollagens in genanntem Medikament zwischen 35 und 55 mg pro Milliliter des Medikaments umfasst.

2. Verwendung von Kollagen, quervernetzt mit einem reduzierenden Zucker nach Anspruch 1, wobei es sich bei dem genannten Zucker um D(-)-Ribose handelt.

3. Verwendung von Kollagen, quervernetzt mit einem reduzierenden Zucker nach einem der vorhergehenden Ansprüche, wobei das genannte Medikament ferner einen pharmazeutisch verträglichen Puffer oder eine pharmazeutisch verträgliche injizierbare Flüssigkeit umfasst.

4. Verwendung von Kollagen, quervernetzt mit einem reduzierenden Zucker nach einem der vorhergehenden Ansprüche, wobei das genannte Kollagen aus der Gruppe ausgewählt ist, die aus Atelopeptid-Kollagen vom Rind, Atelopeptid-Kollagen vom Schwein, Kollagen, gewonnen aus Wirbetierspezies, humanem Kollagen, rekombinantem Kollagen, genetisch hergestelltem oder veränderten Kollagen, Kollagen-Typ I, Kollagen-Typ II, Kollagen-Typ IX, künstlich produziertem Kollagen, hergestellt durch genetisch veränderte eukaryontische oder prokaryontische Zellen oder durch genetisch veränderte Organismen, gereinigtem Kollagen und gereinigtem pepsiniertem Atelopeptid-Kollagen Typ I vom Schwein, Partikel von fibrillärem Kollagen, fibrillär rekonstituiertem Atelopeptid-Kollagen und Kombinationen davon besteht.

5. Verwendung von Kollagen, quervernetzt mit einem reduzierenden Zucker nach einem der vorhergehenden Ansprüche, wobei die genannte Wirbelsäule ausgewählt ist unter einer Wirbelsäule eines Säugetiers, einer Wirbelsäule eines Menschen und einer Wirbelsäule eines Hundes.

6. Verwendung von Kollagen, quervernetzt mit einem reduzierenden Zucker nach einem der vorhergehenden Ansprüche, wobei das genannte Medikament wirksam bei der Wiederherstellung und/oder Erhaltung von mindestens einer mechanischen Eigenschaft der Wirbelsäule oder einem Teil davon ist, wobei die genannte Wiederherstellung und/oder Erhaltung mindestens einen der folgenden Effekte umfasst:
- Vergrößern des Abstands zwischen zwei Wirbeln, die an eine Bandscheibe der genannten Wirbelsäule angrenzen,
- wenigstens teilweise Wiederherstellung der flexiblen mechanischen Eigenschaften einer Bandscheibe der genannten Wirbelsäule,
- wenigstens teilweise Wiederherstellung der torsionsmechanischen Eigenschaften einer Bandscheibe der genannten Wirbelsäule,
- wenigstens teilweise Erhaltung des Abstands zwischen den beiden Wirbeln, die an eine Bandscheibe der genannten Wirbelsäule angrenzen,
- wenigstens teilweise Erhaltung der flexiblen mechanischen Eigenschaften einer Bandscheibe der genannten Wirbelsäule nach der Injektion des genannten Medikaments,
- wenigstens teilweise Erhaltung der torsionsmechanischen Eigenschaften einer Bandscheibe der genannten Wirbelsäule nach der Injektion des genannten Medikaments,
- Induzieren der Bildung von Faserknorpel in vivo in dem inneren Bereich der genannten Bandscheibe oder in dem Nucleus Pulposus einer Bandscheibe der genannten Wirbelsäule,
- Induzieren der Entwicklung von einem oder mehreren Knorpelgewebe(n), Faserknorpelgewebe(n) sowie dichtem fibrösem Gewebe(n) in dem inneren Bereich der genannten Bandscheibe oder in dem Nucleus Pulposus einer Bandscheibe der genannten Wirbelsäule.

7. Verwendung von Kollagen, quervernetzt mit einem reduzierenden Zucker nach einem der vorhergehenden Ansprüche, wobei die genannte Wiederherstellung auch wenigstens zu einer teilweisen Verbesserung der Rückenschmerzen führt.

8. Verwendung von Kollagen, quervernetzt mit einem reduzierenden Zucker nach einem der vorhergehenden Ansprüche, wobei das genannte Medikament ferner einen Inhaltsstoff oder mehrere Inhaltsstoffe enthält, die ausgewählt sind unter nichtquervernetztem Kollagen, Hyaluronsäure, quervernetzter Hyaluronsäure, Hyaluronan, einem Arzneimittel, einer therapeutischen Substanz, einer anästhesierenden Substanz, einer entzündungshemmenden Substanz, Glykosaminoglykanen, Proteoglykanen, morphogenen Proteinen, Glykoproteinen, Mucoproteinen, Mucopolysacchariden, Wachstumsfaktoren, Transkriptionsfaktoren, entzündungshemmenden Substanzen, Proteinen, Peptiden, Hormonen, genetischem Material oder Gentherapie, einer Nukleinsäure, einer chemisch veränderten Nukleinsäure, einem Oligonukleotid, Ribonukleinsäure, Desoxyribonukleinsäure, einem chimären DNA/RNA-Konstrukt, DNA- oder RNA-Sonden, Antisense-DNA, Antisense-RNA. einem Gen, einem Teil eines Gens, einer Zusammensetzung enthaltend natürlich vorkommende oder künstlich hergestellte Oligonukleotide, einer Plasmid-DNA, einer Kosmid-DNA, viralen und nichtviralen Vektoren, die für die Förderung der zellulären Aufnahme und Transkription erforderlich sind, einem Glykosaminoglykan, Chondroitin-4-Sulfat, Chondroitin-6-Sulfat, Keratansulfat, Dermatansulfat, Heparin, Heparansulfat, Hyaluronan, einem lecithinreichen interstitiellen Proteoglykan, Decorin, Biglykan, Fibromodulin, Lumican, Aggrecan, Syndecanen, Beta-Glykan, Versican, Centroglykan, Serglycin, einem Fibronectin, Fibroglykan, Chondroadherinen, Fibulinen, Thrombospondin-5, einem Enzym, einem Enzyminhibitor und einem Antikörper.

9. Verwendung von Kollagen, quervernetzt mit einem reduzierenden Zucker nach einem der vorhergehenden Ansprüche, wobei das genannte Medikament ferner einen Typ oder mehrere Typen an lebenden Zellen enthält.

10. Verwendung von Kollagen, quervernetzt mit einem reduzierenden Zucker nach einem der vorhergehenden Ansprüche, wobei das genannte Medikament ferner einen Typ oder mehrere Typen an lebenden Zellen enthält und wobei die genannten lebenden Zellen ausgewählt sind unter Wirbeltier-Chondrozyten, Wirbeltier-Stammzellen, Wirbeltier-Vorläuferzellen, Wirbeltier-Fibroblasten, Zellen, die genetisch verändert sind, um ein oder mehrere Matrixprotein(e) zu sezernieren, Glykosaminoglykanen, Proteoglykanen, morphogenen Proteinen, Wachstumsfaktoren, Transkriptionsfaktoren, entzündungshemmenden Substanzen, Proteinen, Hormonen, Peptiden und Zellen, die eine Kombination davon exprimieren, wobei es sich bei den genannten Stammzellen nicht um humane embryonale Stammzellen handelt.

11. Verwendung von Kollagen, quervernetzt mit einem reduzierenden Zucker nach einem der vorhergehenden Ansprüche, wobei das das genannte Medikament ferner einen Typ oder mehrere Typen an lebenden Zellen enthält, die so verändert sind, dass sie Rezeptoren für eine oder mehrere der Moleküle exprimieren, die aus der Gruppe ausgewählt sind, die Proteine, Peptide, Hormone, Glykosaminoglykane, Proteoglykane, morphogene Proteine, Wachstumsfaktoren, Transkriptionsfaktoren, entzündungshemmende Substanzen sowie Zellen, die eine Kombination der genannten Rezeptoren exprimieren, enthält.

12. Verwendung von Kollagen, quervernetzt mit einem reduzierenden Zucker nach einem der vorhergehenden Ansprüche, wobei das genannte Medikament ferner einen Typ oder mehrere Typen an lebenden Zellen enthält, wobei diese lebenden Zellen ausgewählt sind unter:
- genetisch veränderten Zellen, die eine oder mehrere Substanz(en) sezernieren, ausgewählt unter Matrixproteinen, Glykosaminoglykanen, Proteoglykanen, morphogenen Proteinen, Wachstumsfaktoren, Transkriptionsfaktoren, entzündungshemmenden Substanzen, Proteinen, Hormonen und Peptiden,
- veränderten Zellen, die Rezeptoren für ein oder mehrere Molekül(e) exprimieren, ausgewählt aus der Gruppe, die aus Proteinen, Peptiden, Hormonen, Glykosaminoglykanen, Proteoglykanen, morphogenen Proteinen, Wachstumsfaktoren, Transkriptionsfaktoren und entzündungshemmenden Substanzen besteht, sowie
- Zellen, die eine Kombination der genannten Rezeptoren und genannten Substanzen exprimieren.

## Revendications

1. Utilisation de collagène réticulé avec un sucre réducteur pour la fabrication d'un médicament liquide injectable pour le traitement d'une pathologie de la colonne vertébrale **caractérisée par** un disque dégénéré, dans lequel la concentration dudit collagène en ledit médicament est comprise entre 35 et 55 mg par millilitre de médicament.

2. Utilisation de collagène réticulé avec un sucre réducteur selon l'une quelconque des revendications précédentes, dans laquelle ledit sucre est du D(-) ribose.

3. Utilisation de collagène réticulé avec un sucre réducteur selon l'une quelconque des revendications précédentes dans laquelle ledit médicament comprend en outre un tampon acceptable sur le plan pharmaceutique ou un fluide injectable acceptable sur le plan pharmaceutique.

4. Utilisation de collagène réticulé avec un sucre réducteur selon l'une quelconque des revendications précédentes, dans laquelle ledit collagène est choisi dans le groupe comprenant du collagène bovin atélopeptide, du collagène porcin atélopeptide, du collagène obtenu à partir d'une espèce vertébrée, du collagène humain, du collagène recombiné, du collagène génétiquement modifié ou recombinante, du collagène de type I, du collagène de type II, du collagène de type IX, collagène produit de façon artificielle fabriqué par des cellules eucaryotiques ou procaryotiques génétiquement modifiées ou par des organismes génétiquement modifié, du collagène purifié et du collagène porcin atélopeptide de type I purifié digéré par enzymes, des particules de collagène fibrillaire, du collagène atélopeptide reconstitué fibrillaire, et des combinaisons de ceux-ci.

5. Utilisation de collagène réticulé avec un sucre réducteur selon l'une quelconque des revendications précédentes, dans laquelle ladite colonne vertébrale est choisie parmi une colonne vertébrale mammifère, une colonne vertébrale humaine et une colonne vertébrale canine.

6. Utilisation de collagène réticulé avec un sucre réducteur selon l'une quelconque des revendications précédentes, dans laquelle ledit médicament est efficace pour restaurer et/ou préserver au moins une propriété mécanique de la colonne vertébrale ou d'une partie de celle-ci, ladite restauration et/ou préservation comprend au moins un des effets suivants :
- augmentation de l'interstice entre les deux vertèbres flanquant un disque intervertébral de ladite colonne vertébrale,
- restauration au moins partielle des propriétés mécaniques flexionnelles d'un disque intervertébral de ladite colonne vertébrale,
- restauration au moins partielle des propriétés mécaniques de torsion d'un disque intervertébral de ladite colonne vertébrale,
- maintien au moins partiel de l'interstice entre les deux vertèbres flanquant un disque intervertébral de ladite colonne vertébrale,
- maintien au moins partiel, après l'injection dudit médicament, des propriétés mécaniques flexionnelles d'un disque intervertébral de ladite colonne vertébrale,
- maintien au moins partiel, après l'injection dudit médicament, des propriétés mécaniques de torsion d'un disque intervertébral de ladite colonne vertébrale,
- déclenchement de la formation in vivo de fibrocartilage dans l'espace interne dudit disque intervertébral ou dans le nucleus pulposus d'un disque intervertébral de ladite colonne vertébrale,
- déclenchement du développement d'un ou de plusieurs tissus cartilagineux, de tissus fibrocartilagineux et de tissus fibreux denses dans l'espace interne dudit disque intervertébral ou dans le nucleus pulposus d'un disque intervertébral de ladite colonne vertébrale.

7. Utilisation de collagène réticulé avec un sucre réducteur selon l'une quelconque des revendications précédentes dans laquelle ladite restauration a également pour conséquence le soulagement au moins partiel de la douleur dorsale.

8. Utilisation de collagène réticulé avec un sucre réducteur selon l'une quelconque des revendications précédentes dans laquelle ledit médicament comprend en outre un ou plusieurs ingrédients choisis parmi du collagène non-réticulé, de l'acide hyaluronique, de l'acide hyaluronique réticulé, de l'hyaluronane, un médicament, un agent thérapeutique, un agent anesthésique, un agent anti-inflammatoire, des glycosaminoglicanes, protéoglycanes, des glycoprotéines de protéines morphogéniques, des mucoprotéines, des mucopolysaccharides, des facteurs de croissance, des facteurs de transcription, des agents anti-inflammatoires, des protéines, des peptides, des hormones, du matériel génétique pour la thérapie génique, un acide nucléique, un acide nucléique chimiquement modifié, un oligonucléotide, un acide ribonucléique, acide désoxyribonucléique, une construction chimérique d'ADN/ARN, des sondes ADN ou ARN, de l'ADN antisens, de l'ARN antisens, un gène, une partie de gène, une composition incluant les oligonucléotides produits de façon naturelle ou artificielle, de l'ADN plasmidique, de l'ADN cosmidique, des vecteurs viraux et non-viraux requis pour favoriser la capture et la transcription cellulaires, un glycosaminoglycane, de la chondroïtine 4-sulfate, de la chondroïtine 6-sulfate, du sulfate de kératane, du dermatane sulfate, de l'héparine, d'héparane sulfate, d'hyaluronane, du protéoglycane interstitiel riche en lécitine, de la décorine, du biglycane, de la fibromoduline, du lumicane, de l'aggrecane, des syndécanes, du bêta-glycane, du versicane, du centroglycane, de la serglycine, de la fibronectine, du fibroglycane, des chondroadhérines, des fibulines, thrombospondine-5, une enzyme, un inhibiteur d'enzyme et un anticorps.

9. Utilisation de collagène réticulé avec un sucre réducteur selon l'une quelconque des revendications précédentes dans laquelle ledit médicament comprend en outre un ou plusieurs types de cellules vivantes.

10. Utilisation de collagène réticulé avec un sucre réducteur selon l'une quelconque des revendications précédentes dans laquelle ledit médicament comprend en outre un ou plusieurs types de cellules vivantes et dans laquelle lesdites cellules vivantes sont choisies parmi des chondrocytes de vertébrés, des cellules souches de vertébrés, des cellules progénitrices de vertébrés, des fibroblastes de vertébrés, des cellules génétiquement modifiées pour sécréter une ou plusieurs protéines de matrice, des glycosaminoglicanes, des protéoglycanes, des protéines morphogènes, des facteurs de croissance, des facteurs de transcription, des agents anti-inflammatoires, des protéines, des hormones, des peptides, et des cellules qui expriment toutes les combinaisons de ceux-ci, dans laquelle lesdites cellules souches ne sont pas des cellules souches embryonnaires humaines.

11. Utilisation de collagène réticulé avec un sucre réducteur selon l'une quelconque des revendications précédentes dans laquelle ledit médicament comprend en outre un ou plusieurs types de cellules vivantes modifiées pour exprimer des récepteurs pour une ou plusieurs molécules choisies dans le groupe comprenant des protéines, des peptides, des hormones, des glycosaminoglicanes, des protéoglycanes, des protéines morphogènes, des facteurs de croissance, des facteurs de transcription, des agents anti-inflammatoires, et des cellules qui expriment toutes les combinaisons desdits récepteurs.

12. Utilisation de collagène réticulé avec un sucre réducteur selon l'une quelconque des revendications précédentes dans laquelle ledit médicament comprend en outre un ou plusieurs types de cellules vivantes et dans laquelle lesdites cellules vivantes sont choisies parmi :
- des cellules génétiquement modifiées pour sécréter une ou plusieurs de substances choisies parmi des protéines de matrice, des glycosaminoglicanes, des protéoglycanes, des protéines morphogènes, des facteurs de croissance, des facteurs de transcription, des agents anti-inflammatoires, des protéines, des hormones et des peptides,
- des cellules modifiées pour exprimer des récepteurs pour une ou plusieurs molécules choisies dans le groupe comprenant des protéines, des peptides, des hormones, des glycosaminoglicanes, des protéoglycanes, des protéines morphogènes, des facteurs de croissance, des facteurs de transcription et des agents anti-inflammatoires, et
- des cellules qui expriment une quelconque combinaison desdits récepteurs et desdites substances.
